# EUROPEAN PATENT APPLICATION

(11) **EP 1 705 186 A1**
(43) Date of publication of application: **27.09.2006**
(21) Application number: 05102298.6
(22) Date of filing: 22.03.2005
(51) Int. Cl.: C07K 14/435, C12N 15/12, C07K 16/18, A61K 39/00, C12N 9/10, C12N 9/02

(54) **Vaccine and antibody against Dictyocaulus viviparus**

(71) Applicant: Stiftung Tierärztliche Hochschule Hannover, 30559 Hannover (DE)
(72) Inventor: Strube, Christina, Dr., 30173 Hannover (DE); von Samson-Himmelstjerna, Georg, Dr., 30900 Mellendorf (DE); Schnieder, Thomas, Prof. Dr., 30539 Hannover (DE)
(74) Representative: Taruttis, Stefan Georg

(57) **Abstract**

The present invention relates to a vaccine to provide immunoprotection against *Dictyocaulus* viviparus in cattle and use of the vaccine to produce specific antibody against *Dictyocaulus* viviparus. The vaccine provides the advantage of eliciting an immune reaction against inhibited and non-inhibited larvae of *Dictyocaulus* viviparus. In a further aspect, the present invention also provides antibody specific for said vaccine.

## Description

The present invention relates to a vaccine against parasitic nematodes of the genus Strongylida, especially to *Dictyocaulus* spp., preferably *Dictyocaulus filaria, Dictyocaulus arnfieldi, Dictyocaulus capreolus, Dictyocaulus cameli, Dictyocaulus africanus,* and most preferably to *Dictyocaulus viviparus.* For the purposes of the invention, reference to *Dictyocaulus* spp. includes the aforementioned species. Further, the invention relates to the use of antigenic peptides for analytical and diagnostic purposes to detect an infection by parasitic nematodes of the genus Strongylida, especially by *Dictyocaulus* spp. and to provide a method for providing immunoprotection against parasitic nematodes of the genus Strongylida, especially *Dictyocaulus* spp.

Further, the present invention relates to a process for producing a vaccine suitable for providing immunity against infections by parasitic nematodes of the genus Strongylida, especially *Dictyocaulus* spp. and for the production of specific antibodies against parasitic nematodes of the genus Strongylida, especially *Dictyocaulus* spp. as well as antibodies, specifically directed against parasitic nematodes of the genus Strongylida, especially *Dictyocaulus* spp.

In greater detail, the present invention provides a combination of antigenic peptides that are immunoreactive components of *Dictyocaulus* spp., especially *Dictyocaulus viviparus* that can be used for producing a vaccine for producing an immune response and antibody directed against parasitic nematodes of the genus Strongylida, especially *Dictyocaulus* spp. and *Dictyocaulus viviparus.*

### State of the art

*Dictyocaulus viviparus,* a bovine lungworm is the cause of parasitic bronchitis impairing the development of infected animals, often leading to death of calves.

*Dictyocaulus viviparus* has a development cycle of a first larval stage, which is secreted by the faeces of infected host animals. Under appropriate climatic circumstances these first stage larvae develop within a week into the second larval stage and a third larval stage, which are infectious larvae.

The infection of cattle is by ingestion of third stage lungworm larvae, which are activated by contact with the gall bile of the host, enabling larvae to pass intestinal mucosa and move through the lymphoid system to the mesenteric lymph nodes, where fourth stage larvae develop by skinning. Larvae of the fourth stage, via passage through the heart reach the lung, where larvae bore into the alveoli. Within the alveoli, larvae skin to develop into pre-adult fifth stage larvae and reach bronchioli and bronchial tubes at about 1 week post infection *(p. i. ).* After an intensive growth phase reproducible adult lung worms occur 3 to 4 weeks *p. i.*

Worm eggs reach the pharynx via the tracheal epithelia and are swallowed by the host animal and a small proportion is coughed out. During the passage through stomach and intestine, first larvae hatch and reach the pasture with faeces. At temperatures below 8 °C in the environment, following ingestion by the novel host, an inhibition of the development occurs in early pre-adult stage larvae.

The inhibition of the development of *Dictyocaulus viviparus* is at least in part caused by climatic conditions which are unfavourable for the development and survival of active stages outside a host, like low temperatures (winter inhibition) or at high temperatures or very dry weather (dry season inhibition).

To-date, a vaccine against *Dictyocaulus viviparus* consists of larvae isolated from the faeces of infected animals, which larvae are attenuated by irradiation at 400 Gray preventing a development to the adult stage. However, production of this vaccine uses the artificial infection of calves to produce larvae secreted in the faeces and is rather costly. Further, a disadvantage of the known live vaccine is its limited shelf life of about four months.

Liddell et al. (Extracellular and cytoplasmic Cu/Zn superoxide dismutases from Haemonchus contortus, Parasitology 116, 383-394 (1998)) cite the immunological importance of SOD from *Dictyocaulus viviparus* for the immunization of cattle. In addition, a combination of cytosolic and extracellular Cu/Zn superoxide dismutases is used as a vaccine against *Haemonchus contortus.*

For paramyosin, full-length and fragmented paramyosin from *Taenia solium* have been described in detail as a possible vaccine against cysticercosis (Vazquez-Talavera et al., Characterisation and protective potential of the immune response to Taenia solium in a murine model of cysticercosis, Infection and Immunity, 5412-5416 (2001)). However, the present inventors have found that the paramyosin gene of *Taenia solium* has only limited homology to the newly identified paramyosin gene *of Dictyocaulus viviparus.*

In contrast to the suggested suitability of paramyosin from *Taenia solium* as a possible vaccine against schistosoma and filaria, Pearce et al. (Induction of protective immunity against Schistosoma mansoni by vaccination with schistosome paramyosin (Sm97), a nonsurface parasite antigen, PNAS 85, 5678-5682 (1988)) demonstrated that paramyosin from *Mercenaria mercenaria,* a heterologous paramyosin to the one from *Schistosoma mansoni,* did not induce a protective immune response in mice.

Another paramyosin that has been suggested for use as an antigen against a parasitic infection is paramyosin of *Brugia malayi* (Nanduri et al., The Journal of Immunology, 143, 3359-336 (1989) Paramyosin enhanced clearance *of Brugia malayi* microfilaremia in mice). Although classified as a nematode, *Brugia,* living in the blood of its human hosts, do not belong to the stem of Strongylida, and have a different life cycle from Strongylida.

A further hint for the importance of paramyosin for immunoprotection against *Brugia malayi* comes from Li et al., The Journal of Immunology 150, 1881-1885 (1993) analyzing the immunoprotection obtained after immunization with irradiated larvae or with paramyosin of *Brugia malayi.*

McManus et al. (A vaccine against Asian schistosomiasis, Parasitology International 53, 163-173 (2004)) relate to the potential use of paramyosin as an antigenic compound obtainable from *Schistosoma japonicum* to provide protective immunity against several Schistosoma. McManus et al. preferably relate to regions of high homology of paramyosins from *Schistosoma.*

Investigating paramyosin as a candidate vaccine antigen against the human parasite *Schistosoma japonicum,* Ramirez et al. (Parasite Immunology 18, 49-52 (1996)) could show that the schistosome myosin as well as paramyosin from the nematode *Mercenaria* did not induce immunoprotection against *Schistosoma japonicum.* The fmding that vaccination with paramyosin from *Schistosoma japonicum* in contrast to paramyosin from *Schistosoma mansoni* elicits a better protective immune response indicates that the origin of the paramyosin determines its specificity for the respective parasite species. In accordance with suggestions by Ramirez et al. to test the immunoprotective potential elicited by paramyosin from one strain of *Schistosoma japonicum* even against different strains, it is reasonable to assume that the immunologic importance of paramyosin as an antigen for protective immunity is limited to the specific Schistosome species, possibly even to the specific strain under investigation.

In contrast to the state of art making reference to paramyosin obtained from a certain species of trematodes and cestodes, the present invention refers to paramyosin obtainable from *Dictyocaulus viviparus,* belonging to the class of Strongylida, for use as an antigenic vaccine component to elicit an immunoreaction against Strongylida, preferably against *Dictyocaulus* spp.. It is known that the parasites reported to be under investigation, e.g. *Taenia solium, Schistosoma mansoni, Schistosoma japonicum* and *Brugia malayi* each have very different development cycles infect different hosts and cause different diseases from Strongylida, especially *Dictyocaulus viviparus.*

The immunoreactivity of SOD of *Dictyocaulus viviparus* is known from Britton et al. (Superoxide dismutase (SOD) activity of Dictyocaulus viviparus and its inhibition by antibody from infected and vaccinated bovine hosts, Parasitology, 109, 255-261 (1994)). SOD activity was detected in PBS soluble extracts of L3 and adult stages of *Dictyocaulus viviparus* as well as in excretory secretory products of adult stages. When analyzing SOD by native polyacrylamide gel electrophoresis, different distributions of isoenzymes were found for adult extract in comparison to excretory secretory products. The excretory secretory isoenzyme of SOD was demonstrated to be antigenic by immune reaction with IgG antibody obtained from serum of infected or vaccinated bovine hosts.

However, the present invention refers to a defined SOD, namely the extracellular SOD, for use as a vaccine component.

### Objects of the invention

It is an object of the present invention to provide a vaccine composition effectively providing immunoprotection against the parasites of the genus Strongylida, especially against *Dictyocaulus viviparus.* Due to the simultaneous occurrence of inhibited and non-inhibited larval stages of *Dictyocaulus viviparus,* it is a preferred object of the present invention to provide a vaccine composition that provides immunoprotection against both inhibited and non-inhibited stages of the lungworm.

It is also an object of the present invention to provide a vaccine effective against Strongylida, especially against *Dictyocaulus viviparus,* which can be produced without artificial infection of host animals, preferably resulting in a vaccine having improved stability during storage.

It is a further object of the present invention to provide antibody reactive with both inhibited and non-inhibited stages of Strongylida, especially *of Dictyocaulus viviparus.* Such antibody can be used for diagnostic purposes to detect an infection in ruminants, especially in cattle, independent of the development stage of Strongylida, especially *Dictyocaulus viviparus.* As a further application of such antibody, passive immunization is desired to provide passive immunity or to support the immune defence of the host against Strongylida, especially *Dictyocaulus* spp..

As a further object, the present invention seeks to provide a method for producing antigenic protein for use in vaccines eliciting protection against Strongylida, especially *Dictyocaulus* spp.

### General description of the invention

In general, the present invention provides a vaccine capable of eliciting an immune response in cattle against Strongylida effective to reduce the excretion of larvae from infected animals and to reduce the worm burden of infected animals. Accordingly, immunization of cattle with a vaccine according to the present invention reduces the effects of an infection with Strongylida, especially *Dictyocaulus* spp., preferably up to full protection against infection, for example protection against the re-infection syndrome.

In a first embodiment, the present invention provides a vaccine suitable for providing immunoprotection against Strongylida, especially *Dictyocaulus* spp., comprising protein containing immunogenic epitopes of at least one of phenyl ethanolamine-N-methyltransferase (PNMT), paramyosin, and extra-cellular copper-zinc superoxide dismutase (SOD), all obtainable from the respective genes of Strongylida, especially *Dictyocaulus* spp..

The epitopes of the vaccine can be provided in the form of complete or partial translation products of the respective genes encoding PNMT, paramyosin, and SOD, respectively. Further, fusion peptides can be used to provide epitopes of one or a combination of the original genes. Such fusion peptides can be translated from synthetically produced nucleic acid sequences encoding a non-natural combination of epitopes derivable from the genes encoding PNMT, paramyosin and SOD. Epitopes can be identified according to standard practice, e.g. by computer-assisted analysis for immunogenic protein regions and/or by testing the immunogenicity of protein portions.

Accordingly, in the following description, the terms SOD, PNMT and paramyosin refer to the expression products of the respective genes obtainable from Strongylida, especially from *Dictyocaulus viviparus,* and derivatives thereof having immunoreactivity, e.g. peptides comprising epitopes of the aforementioned gene products which are immunoreactive at least in cattle.

It was also shown that a combination of epitopes from at least two, preferably three, peptides from the group of PNMT, SOD, and paramyosin resulted in a stronger immunoprotection than a vaccine comprising the epitopes of only one of the aforementioned immunogenic proteins. Accordingly, it is preferred that the vaccine comprises epitopes of at least two of PNMT, SOD, and paramyosin obtainable from the Strongylida, preferably from *Dictyocaulus viviparus.* Even more preferred, the vaccine comprises PNMT and one or both of SOD, and paramyosin in order to provide protection against both inhibited and non-inhibited stages of Strongylida, especially *Dictyocaulus viviparus.*

The vaccine compositions can be used for the treatment of seronegative and/or infected animals.

In addition to eliciting an antibody response, it is preferred to provide immunoprotection in ruminants, especially in cattle against Strongylida, especially *Dictyocaulus* spp. by eliciting a cellular immune response. In accordance with analyses of cattle immunized with vaccine compositions according to the present invention, it has been found that a significant reduction of the worm burden of *Dictyocaulus viviparus* after artificial infection could be reached in comparison to non-vaccinated control animals. However, the reduction of the worm burden could not only be assigned to antibody titers and, accordingly, it is assumed that the vaccine compositions are able to elicit a cellular immune response in cattle.

In one aspect of the first embodiment, the present invention is focused on the immunoreactivity of paramyosin obtainable from Strongylida, especially *Dictyocaulus viviparus.* Although Strongylida belong to a different stem of helminths than the genera investigated in the known state of art for their immunoreactivity in respect of their specific paramyosin, the present inventors have found that the paramyosin obtainable from Strongylida, especially paramyosin of *Dictyocaulus viviparus* is a suitable antigen for eliciting an at least partially protective immune response in ruminants, especially in cattle.

In a further aspect of the first embodiment, the present invention is based on the fmding that a combination of paramyosin and SOD obtainable from *Dictyocaulus viviparus,* which are both predominantly expressed in the non-inhibited larval stage *of Dictyocaulus viviparus,* can induce an improved immunoreaction in cattle, preferably sufficient to provide immunoprotection against infection by Strongylida, preferably *Dictyocaulus* spp..

In its preferred embodiment, the first embodiment of the present invention is based on the fmding that PNMT is predominantly expressed, i.e. is characteristic for inhibited larval stages at least of *Dictyocaulus viviparus* and elicits an immune reaction in ruminants, whereas paramyosin and SOD are preferentially expressed in Strongylida, especially in *Dictyocaulus viviparus,* during their non-inhibited stages.

Therefore, it is preferred that the vaccine contains epitopes of PNMT in combination with at least one of SOD and paramyosin, preferably in combination with at least paramyosin, obtainable from Strongylida, especially from *Dictyocaulus viviparus.* When present in combination with at least one of SOD and paramyosin, the vaccine comprising PNMT is more effective to elicit an immune response in cattle against non-inhibited and inhibited stages of Strongylida, especially against *Dictyocaulus* spp..

In addition to epitopes of PNMT in combination with SOD and/or paramyosin, the vaccine can comprise epitopes of acetylcholine esterase (ACE), major sperm protein (MSP) and/or protein disulfide isomerase (PDI) obtainable from Strongylida, especially from *Dictyocaulus viviparus.* The epitopes derivable from ACE, MSP and PDI may be present in the vaccine in the form of the translation products of the respective natural gene sequences, or as partial translation products or as portions of a fusion peptide as described for PNMT, SOD and paramyosin. Such a fusion peptide may comprise specific epitopes in a new, i.e. non-natural arrangement.

In addition to the immunogenic peptides mentioned hereinabove, the vaccine according to the invention may comprise adjuvants, e.g. Alum, more preferred Quil A, and further known additives like preservatives and antioxidants.

Further, the present invention provides a method for producing vaccine compositions suitable for eliciting a partial, preferably a complete immunoprotection against Strongylida, e.g. against *Dictyocaulus* spp., which method is characterized by providing the specific immunoreactive proteins of the invention. For production, heterologous expression of each of PNMT, paramyosin and/or SOD, optionally each of ACE, MSP and PDI, each of the aforementioned peptides as complete or partial translation products of the natural respective genes in a microorganism is preferred. For heterologous expression and subsequent purification steps of the immunoreactive proteins, nucleic acid sequences encoding amino acid sequences may be added or deleted, for example a tag for affinity purification like a poly-histidine sequence may be added for ease of purification, or non-immunogenic regions may be deleted. Modifications of the immunoreactive proteins of the invention can be practised as long as the immunoreactive properties remain essentially unchanged, e.g. as long as the specific epitopes of PNMT, paramyosin, and/or SOD optionally of ACE, MSP and/or PDI are not impaired with respect to their antigenicity.

In a second embodiment, the present invention provides antibody preparations directed against the antigenic proteins of the vaccine compositions according to the first embodiment. Such antibody preparations are specific for at least one of PNMT, SOD and paramyosin or, alternatively, specific for two or all three of paramyosin, SOD, and PNMT, preferably including specificity against PNMT in combination with at least one of SOD and PNMT. Accordingly, these antibody preparations are immunoreactive with both protein expressed in the inhibited state as well as with protein expressed in the non-inhibited state of *Dictyocaulus* spp..

These antibody compositions can be used to provide passive immunization of host animals against Strongylida, especially against *Dictyocaulus viviparus,* e.g. for treatment of seronegative and/or infected animals. Further, these antibody compositions can be used for analytical purposes, i.e. for detecting an infection of ruminants by Strongylida like *Dictyocaulus viviparus.*

### Detailed description of the invention

The examination of preferential gene transcription of third stage larvae in an inhibited (L3i) and a non-inhibited (L3ni) stage of *Dictyocaulus viviparus* demonstrated that PNMT, SOD and paramyosin are differentially expressed in third stage larvae, with PNMT characteristic for the inhibited stage and SOD as well as paramyosin characteristic for the non-inhibited stage, respectively. In accordance with the differential expression, the genes are transcribed differentially according to the status of the inhibition.

An immunological reaction in ruminants against each of the proteins PNMT, SOD, and paramyosin was shown by antibody production in response to immunization with each of these proteins, or a combination thereof, in calves originally having a negative immune status to *Dictyocaulus viviparus.* Further to the induction of an immune response against PNMT, SOD and/or paramyosin in a calf, a specific immune reaction could also be induced by immunizing ruminants, for example cattle, with immunoreactive portions of said proteins, e.g. synthetic peptides or comprising epitopes eliciting a specific antibody response.

Vaccination was done by i.m., i.v., i.p. injection of the peptides in admixture with a suitable adjuvant, e.g. Quil A. However, other routes of administration can be chosen, for example oral administration.

In addition to antibody production, the immune response also included a cellular immune response, which for example can be demonstrated in lymphocyte proliferation assays. The effectiveness of a vaccine according to the invention is evident when comparing infected animals without or after prior immunization with the vaccine, showing on the one hand a reduction of the number of *Dictyocaulus viviparus* larvae shed with the faeces by a factor of at least 2, and on the other hand a reduction of the worm burden within the lungs of infected calves. Analysis of this comparison also demonstrated that vaccination significantly reduced the number of both non-inhibited and of inhibited *Dictyocaulus viviparus* larvae as counted from the lungs of sacrificed animals.

For the production of an immune response specific for parasitic nematodes, preferably of the genus Strongylida, preferably against *Dictyocaulus* spp, host animals are immunized with a vaccine according to the present invention using an immunization strategy, for example using a boosting strategy, according to standard procedures.

For analytical purposes, serum from immunized animals can be used to specifically detect parasitic nematodes, for example *Dictyocaulus viviparus,* independent from their condition of an inhibited or non-inhibited stage, e.g. using an antibody preparation linked to a detectable label.

Antibody can be purified according to known procedures. Suitable host animals for producing antibody are for example ruminants, like cattle and sheep, or mice, rats and rabbits. Apart from polyclonal antibody preparations, monoclonal antibody can be produced according to standard procedures, for example using the hybridoma technique on spleen cells obtained from immunized host animals. Monoclonal antibody can be identified for its specificity against either of PNMT, SOD, paramyosin, MSP, ACE or PDI. Production of monoclonal antibody can be performed according to known procedures. According to the invention, it is preferred that monoclonal antibody specific for PNMT is combined with at least one of antibody specific for SOD or antibody specific for paramyosin in order to detect both inhibited and non-inhibited Strongylida.

Polyclonal antibody, e.g. serum from immunized animals, or monoclonal antibody was used for analytical purposes to determine the presence of parasitic nematodes, namely *Dictyocaulus viviparus* in the host animal cattle. For this purpose, a sample of body fluid or a washing from an animal suspected of infection with *Dictyocaulus viviparus,* was homogenized and analysed in a specific immune reaction, generally using an ELISA format. Polyclonal antibody preparations or a combination of monoclonal antibody specific for PNMT with at least one antibody specific for SOD and an antibody specific for paramyosin was used for passive immunization of cattle, e.g. calves to provide at least a partial immunoprotection against subsequent infection with *Dictyocaulus viviparus* through ingestion. Improved results were found for antibody preparations that additionally contained antibody against MSP, optionally also antibodies against ACE and/or PDI.

It is a specific advantage of the present invention that a vaccine composition comprising both PNMT and at least one of SOD and paramyosin provides immunoprotection against inhibited and non-inhibited parasitic nematodes, especially their larvae, preferably against *Dictyocaulus viviparus.* Accordingly, an immune response can be elicited in vaccinated host animals that is also directed against the inhibited larvae of *Dictyocaulus viviparus,* resulting in a reduction or removal of inhibited larvae, which is suitable for interruption of the life cycle of said parasitic nematode. This is regarded a specific advantage because for example adult cattle may be infected by inhibited larvae of *Dictyocaulus viviparus* without showing symptoms i.e. they are not recognized as a source for contamination of pastures with infective eggs or larvae.

For an alternative use of antibody, in diagnostic applications, specific antibodies against each of PNMT, paramyosin, SOD, ACE, MSP, and PDI are provided by the present invention. Both the analytical antibodies and antibody used for treatment of animals can be polyclonal or monoclonal. For both polyclonal and monoclonal antibody to be used for the treatment of animals, e.g. for therapeutic purposes, it is preferred that their constant chains are in accordance with the animal species to be treated, e.g. an immunoreaction by the treated animal against the antibody according to the invention is avoided.

The present invention is now described in greater detail by way of examples.

### Example 1: Analysis of differential gene expression in hypobiosis induced (inhibited) and non-induced (non-inhibited) third-stage larvae of Dictyocaulus viviparus

Generally, suppression subtractive hybridization, followed by differential screening of subtractive cDNA libraries was used to identify differentially expressed genes based on poly(A)⁺RNA of hypobiosis induced (L3i) and non-induced (L3ni) third-stage *Dictyocaulus viviparus* larvae.

To isolate poly(A)⁺ RNA, the exsheathed larvae were homogenized with a mortar and pestle in liquid nitrogen. Poly(A)⁺ RNA was extracted using oligo(dT) cellulose beads from the Quick Prep™ Micro mRNA Purification Kit (Amersham Biosciences). The poly(A)⁺RNA yield was determined by measuring the absorbance at 260 nm. Poly(A)⁺ RNA of the two larval populations was transcribed into double stranded cDNA by using the SMART™ PCR cDNA Synthesis Kit (BD Clontech) according to the manufacturer's recommendations.

Suppression subtractive hybridization was performed with the PCR-Select™ cDNA Subtraction Kit (BD Clontech) according to the manufacturer's protocol. Briefly, following restriction-enzyme digestion of the cDNAs and adaptor ligation of the testers, two rounds of subtraction were done. In the forward subtraction, L3i-cDNA served as tester containing up-regulated transcripts. This tester was hybridized with driver cDNA of the L3ni to remove common gene sequences. In a reverse subtraction the L3ni cDNA served as tester and was hybridized with L3i cDNA as the driver. The subtracted, differentially expressed sequences of both larval populations were amplified using 27 primary and 12 secondary PCR cycles. To examine the efficiency of subtraction, subtracted and unsubtracted secondary PCR products of the L3i and L3ni were amplified using primers for an unspecified gene sequence known to be expressed in both larval populations. The sequences of the primers were 5'-ACG CGT TTA GCA CTA CTG GTT GT-3' (Seq.-ID No 1) and 5'-GGA CGG AAG CTG CTA CAA-3' (Seq.-ID No 2). PCR cycling was performed using the following temperature profile: denaturing at 94 °C for 30 s, annealing at 56 °C for 30 s, and extension at 68 °C for 2 min.

To generate subtracted libraries, the secondary PCR products of the L3i and L3ni generated by suppression subtractive hybridization were directly cloned into the vector pCR™ 4-TOPO™ and transformed into E. *coli* strain One Shot^{TM} TOP10 by the TOPO TA Cloning^{TM} Kit for Sequencing (Invitrogen). Before generating subtracted libraries, randomly selected clones of the L3ni and L3i were checked for inserts using the nested primers of the secondary PCR.

The inserts of subtracted libraries were analyzed for differential expression by differential screening. Therefore, subtracted and unsubtracted primary PCR products of the L3i and L3ni were labeled with DIG-dUTP. Labeling of the cDNAs was done by secondary PCR with a DIG-dUTP:dTTP ratio of 1:6 using the DIG DNA Labeling Mix (Roche), purified with CHROMA SPIN™-100 Columns (BD Clontech), digested with *Rsa*I*, Sma*I*,* and *Eae*I (Roche) to remove adaptors and purified again with CHROMA SPIN™-100 Columns.

The inserts of the subtracted libraries were amplified and arrayed in duplicate onto four positively charged nylon membranes (Roche) according to the protocol of the PCR-Select™ Differential Screening Kit (BD Clontech). The membranes were enclosed in a hybridization bag (Roche) and prehybridized with DIG Easy Hyb solution (Roche) containing 50 µl blocking solution (PCR-Select™ Differential Screening Kit, BD Clontech), and denatured at 99 °C for 10 min before quenching on ice. Prehybridization was performed at 42 °C for 90 min in a water bath with gentle shaking. Of each DIG-labeled probe 495 ng was denatured together with 50 µl blocking solution and added to 30 ml DIG Easy Hyb (16.5 ng probe/ml). This hybridization-solution was added to the membranes. Hybridization was done at 42 °C for 22 h in a water bath with gentle shaking followed by washing twice at RT (10 min/wash) in 2x SSC, 1% SDS and twice at 68 °C in 0.5x SSC, 0.1 % SDS (20 min/wash). The chemiluminescent detection was performed using the DIG High Prime Labeling and Detection Starter Kit II (Roche) according to the manufacturer's recommendations. Following exposure of the membranes to KODAK® BioMax™ Light films (Amersham Biosciences) at RT for 40 min, the films were developed using KODAK® GBX Developer and KODAK® GBX Replenisher (Integra).

To confirm the results of the differential screening procedure, a further cDNA hybridization was performed. Briefly, the amplified inserts of the subtracted libraries were arrayed again in duplicate onto two positively charged nylon membranes (Roche). Freshly isolated poly(A)⁺ RNA (Quick Prep™ Micro mRNA Purification Kit, Amersham Biosciences) of the L3i and L3ni was used to generate DIG-labeled cDNA using the SMART™ PCR cDNA Synthesis Kit (BD Clontech) according to the manufacturer's instructions. The probes were then labeled with a DIG-dUTP:dTTP ratio of 1:6 during LD PCR. To remove the SMART II oligonucleotides, the probes were purified with CHROMA SPIN™-100 Columns (BD Clontech) according to the manufacturer's recommendations, digested with *Rsa*I (Roche), and purified again with CHROMA SPIN™-100 Columns. Preparation of the membranes, hybridization, and detection were carried out as described above with the following modifications: 25 ng probe/ml DIG Easy Hyb (Roche) were used and membranes were exposed for 90 min to the chemiluminescence films.

### Example 2: Sequencing of differentially expressed genes

Plasmid DNA of the clones containing differentially expressed cDNAs were obtained using the Plasmid Midi Kit (Qiagen) and sequenced at the SEQLAB Sequence laboratories (Göttingen, Germany). The resulting sequence data were analyzed for identities to genes and proteins deposited in the NCBI database using the BLAST search program (available at http://www.ncbi.nlm.mh.gov/BLAST/).

As a result of suppression subtractive hybridization according to example 1, it was found that it is PNMT which is predominantly expressed in the inhibited stage of *Dictyocaulus viviparus,* whereas SOD and paramyosin are the proteins predominantly expressed in its non-inhibited stage.

### Example 3: Generating full-length cDNA sequences

To obtain full-length sequences of PNMT, SOD and paramyosin, rapid amplification of cDNA ends (RACE) was carried out using the SMART™ RACE cDNA Amplification Kit (BD Clontech). Briefly, poly(A)⁺RNA of the two larval populations was converted into first-strand cDNA and subsequently amplified using touchdown PCR. In 3'-RACE the following gene specific primers were used:
5'-CACAAAATCGCCAAGAGCTAATGCGGTGGTATGA-3' (Seq.-ID No 3) for PNMT,
5'-GGCAAACGGTTGTCTAGCAGCTGGAGGTCATTATA-3' (Seq.-ID No 4) for SOD,
and 5'-CTTGCCGATGCGAATCGAAAACTGCATGAGTT-3' (Seq.-ID No 5) and
5'-AAGCTAGTTTGGAAGATACCCAACGTCAGCTCCAACAAG-3' (Seq.-ID No 6), for paramyosin respectively.

The following gene specific primers were used in 5'-RACE:
5'-ATTTCGCGTATCACTGTGCTGCCTCAACACCAC-3' (Seq.-ID No 7) for PNMT,
5'-ATACGTATCGCGCCCAATTCCAAGCGTTTAGTTTA-3' (Seq.-ID No 8) for SOD,
and 5'-AGCGGTCGATTTCAAATTGCATATTCTTGCGAAGAG-3' (Seq.-ID No 9), and
5'-GAATAGCGATGGTGTTCTGAGCTTTCTCAAGGTCAACAATAAG-3' (Seq.-ID No 10) for paramyosin, respectively.

Candidate bands were cut from the gel and ligated into pCR™ 4-TOPO™ vector (TOPO TA Cloning™ Kit for Sequencing, Invitrogen) followed by sequencing. Full-length cDNA sequences were obtained by overlapping fragments and subsequently compared to genes, proteins, and conserved domains deposited in the NCBI database using the BLAST search program to confirm that the full-length sequences are PNMT, SOD and paramyosin. For PNMT, five isotypes were identified, for SOD two isotypes. The sequences given are cDNA sequences, from which the respective amino acid sequences were translated. It is assumed that the number of isotypes is caused by post-transcriptional processing, e.g. by alternative splicing.

The sequence listings comprise

| for PNMT | nucleic acid sequence | amino acid sequence |
|---|---|---|
| Isotype 1 | Seq.-ID No. 11 | Seq.-ID No. 16 |
| Isotype 2 | Seq.-ID No. 12 | Seq.-ID No. 17 |
| Isotype 3 | Seq.-ID No. 13 | Seq.-ID No. 18 |
| Isotype 4 | Seq.-ID No. 14 | Seq.-ID No. 19 |
| Isotype 5 | Seq.-ID No. 15 | Seq.-ID No. 20; |

| for SOD | nucleic acid sequence | amino acid sequence |
|---|---|---|
| Isotype 1 | Seq.-ID No. 21 | Seq.-ID No. 23 |
| Isotype 2 | Seq.-ID No. 22 | Seq.-ID No. 24; |

| for paramyosin | nucleic acid sequence | amino acid sequence |
|---|---|---|
| | Seq.-ID No. 25 | Seq.-ID No. 26; |

| for (MSP) | nucleic acid sequence | amino acid sequence |
|---|---|---|
| | Seq.-ID No. 27 | Seq.-ID No. 28; |

| for ACE | nucleic acid sequence | amino acid sequence |
|---|---|---|
| | Seq.-ID No. 29 | Seq.-ID No. 30; |

and

| for PDI | nucleic acid sequence | amino acid sequence |
|---|---|---|
| Isotype 1 | Seq.-ID No. 31 | Seq.-ID No. 33 |
| Isotype 2 | Seq.-ID No. 32 | Seq.-ID No. 34 |

### Example 4: Production of PNMT, SOD and paramyosin by heterologous expression

From each protein, the entire coding region with the exception of the start codon was ligated into pGEX-2T, pGEX-4T-3, pGEX-6P-3 (Amersham Biosciences), followed by PCR for incorporation of a *Bam*HI restriction site at the 5'-end and *Not*I (PNMT and paramyosin) and an *Eco*RI (SOD) restriction sites, respectively, at their 3'-ends. Primers used for PNMT were (5'-GGATCCGAGTCAGCAGACGGTG-3', Seq.-ID No 35), PNMT rev (5'-GCGGCCGCAACAACAGAGTAGAATTAGGAAGTA-3', Seq.-ID No 36), for SOD (5'-GGATCCATACTCCACATTTCACTC -3', Seq.-ID No 37), SOD rev (5'-CTTAAGC CAATT CCAAGCGTTTAGTTTAGA-3', Seq.-ID No 38), for paramyosin (5'-GGAT CCTCTGCTAACTTGTACAG-3', Seq.-ID No 39), and paramyosin rev (5'-GCGGC CGCTTTTGCG GTCACCTTAGAAATC-3', Seq.-ID No 40). Resultant constructs were expressed in BL21 Star™ (DE3) One Shot® chemically competent *Escherichia coli* (Invitrogen). The bacteria were grown to mid-log phase and isopropyl-beta-D-thiogalactoside (IPTG) was added to induce expression of PNMT, SOD and paramyosin, respectively. The cultures were incubated with vigorous agitation at 37 °C. The bacteria were harvested by centrifugation. After lysis of the cells, the expressed proteins containing a vector based GST-tag were purified using the MagneGST™ Protein Purification System (Promega) according to the manufacturer's recommendations and analyzed by sodium dodecyl sulfate-polyacrylamide gel electrophoresis.

### Example 5: Active immunization of cattle for protective immunity against Dictyocaulus viviparus

Worm-free calves younger than six month at the start of the experiment were allocated into three groups of four each. Four calves were immunized three times intramuscularly in the neck at three week intervals with 200 µg recombinant protein according to Example 4 emulsified in 4 mg adjuvant (Alum) and Tris-buffer (10 mM, pH 7.4) up to 1.5 ml. Another four calves were immunized three times intramuscularly in the neck at three week intervals with 200 µg recombinant protein according to Example 4, emulsified in 750 µg Quil A as adjuvant and Tris-buffer (10 mM, pH 7.4) up to 1.5 ml. Control injections (four calves each) were prepared in the same way except with Elution buffer contained in the MagneGST™ Protein Purification System (Promega) replacing the antigen. All calves were challenged three weeks after the final injection by artificial infection with *Dictyocaulus viviparus* and necropsied four weeks later. Animals were observed for clinical normality every day with careful attention on clinical signs of lungworm infection. Faecal larvae counts were made at the days of injection, the day of challenge, and daily for the 21 days after challenge until necropsy. After necropsy, parasites were washed out of the lungs thoroughly to calculate fmal worm burden. Furthermore, the parasites were determined to sex and developmental stage where possible and measured in length.

This protection trial was done with each of PNMT, SOD and paramyosin only, combinations of PNMT and SOD only, PNMT and paramyosin only, SOD and paramyosin, and PNMT combined with both SOD and paramyosin.

The results show that immunization with a vaccine containing only PNMT predominantly reduces the number of inhibited larval stages of *Dictyocaulus viviparus,* whereas vaccination with a vaccine comprising SOD or paramyosin only predominantly reduces the worm burden of non-inhibited larval stages of *Dictyocaulus viviparus.*

Interestingly, later trials using a combination of PNMT and paramyosin or a combination of PNMT and SOD resulted in a stronger reduction of the worm burden of calves, both of inhibited and non-inhibited larval stages of *Dictyocaulus viviparus.* Improved results were determined for vaccine compositions comprising PNMT in combination with both SOD and paramyosin.

In a current experiment (to date one week after the last injection) with MSP, the major sperm protein of *Dictyocaulus viviparus,* used in combination vaccine containing PNMT, paramyosin and SOD, vaccinated animals show a significant increase in the titers of IgGl and IgG2 against the major sperm protein beginning between day four and seven after the first injection compared to the control animals. The worm burden was reduced by a factor of at least 2 in comparison to animals vaccinated with a vaccine containing PNMT, paramyosin and SOD but without MSP.

As an alternative to MSP or in addition to MSP, the addition of ACE and/or PDI to a vaccine comprising a combination of PNMT, paramyosin and SOD resulted a further reduction of the worm burden.

### Example 6: Epitopes from PNMT, SOD and paramyosin to immunize cattle against Dictyocaulus viviparus

Epitopes, which are small regions of a protein molecule that the immune system recognizes and responds to, can be identified and localized in PNMT, SOD and paramyosin, respectively. This was done according to known methods using an epitope mapping technology, for example proteolytic digestion of proteins bound to immobilised antibodies, combined with matrix assisted laser desorption mass spectrometric identification of the remaining affinity-bound peptides. After identification and synthesis these peptides were used for immunization of cattle against *Dictyocaulus viviparus* in a protection treatment comparable to example 5, yielding comparable reductions in worm burden.

The vaccination with epitopes instead of proteins can be advantageous to reduce the allergenic potential, which may be accompanied by atypical or unspecific immune responses.

### Example 7: Production of antibodies to PNMT, SOD or paramyosin

The present invention also relates to the use of PNMT, SOD and paramyosin as antigens to produce polyclonal antibodies or monoclonal antibodies that bind to or neutralize *Dictyocaulus viviparus.* Illustrative protocols for producing these antibodies are described for example by Harlow and Lane (Antibodies: A Laboratory Manual, Cold Spring Harbor Laboratory Press, Cold Spring Harbor, New York, USA (1988)) or Goding (Monoclonal antibodies. Principles and Practice, Academic Press, San Diego, California, USA (1996)).

Inoculum for polyclonal antibody production typically is prepared by dispersing the antigen in a physiologically tolerable diluent such as saline, to form an aqueous composition. An immunostimulatory amount of inoculum, with or without adjuvant, is administered to a mammal, and the inoculated mammal then is maintained for a time period sufficient for the antigen to induce protecting *anti-Dictyocaulus viviparus* antigen antibodies. Boosting doses of the antigen may be used in individuals that are not already primed to respond to the antigen. The antibodies can be harvested and isolated to the extent desired by well known techniques, such as by alcohol fractionation and column chromatography, or by immunoaffmity chromatography; that is, by binding antigen to a chromatographic column packing like Sephadex™, passing the antiserum through the column, thereby retaining specific antibodies and separating out other immunoglobulins (IgGs) and contaminants, and then recovering purified antibodies by elution with a chaotropic agent, optionally followed by further purification.

Polyclonal antibodies were generated against PNMT, SOD or paramyosin only and combinations of these proteins (PNMT with SOD only, PNMT with paramyosin only, PNMT with both SOD and paramyosin, and SOD with paramyosin) by immunization with the entire protein or protein fragments or fusion peptides containing antigenic epitopes.

Generally a monoclonal antibody composition contains only one species of antibody binding to an epitope. Suitable antibodies in monoclonal form to PNMT, SOD or paramyosin can be prepared using conventional hybridoma technology. To form hybridomas from which a monoclonal antibody composition of the present invention is produced, a myeloma or other self-perpetuating cell line is fused with lymphocytes obtained from peripheral blood, lymph nodes or the spleen of a mammal hyperimmunized with the antigen. It is preferred that the myeloma cell line be from the same species as the lymphocytes. Spleenocytes are typically fused with myeloma cells using polyethylene glycol 1500. Fused hybrids are selected by their sensitivity to HAT. Hybridomas secreting the antibody molecules of this invention can be identified using an ELISA. Initiating a monoclonal hybridoma culture comprising a nutrient medium containing a hybridoma that secretes antibody molecules of the appropriate specificity can produce a monoclonal antibody composition of the present invention. The culture is maintained under conditions and for a time period sufficient for the hybridoma to secrete the antibody molecules into the medium. The antibody-containing medium is then collected. The antibody molecules then can be isolated further by well-known techniques.

The monoclonal and polyclonal antibody compositions produced can be used in passive immunization to provide an immune reaction for the prevention or treatment of infection by *Dictyocaulus viviparus.* In this regard, the antibody preparation can be a polyclonal composition. Alternatively, an "engineered oligoclonal" mixture may be used, which is a mixture of monoclonal antibodies.

### Example 8: Diagnostic assays of bovine samples for presence of Dictyocaulus viviparus antigens or specific antibodies against these

The nucleic acid molecules encoding PNMT, SOD and paramyosin, as well as the proteins themselves and antibodies directed against these can be used as diagnostic reagents to detect infection by Strongylida, specially by *Dictyocaulus viviparus.*

An example for detecting presence or absence of mRNA or genomic DNA in a biological sample (e.g. faeces) is reverse transcription and amplification using polymerase chain reaction (PCR), respectively. These methods involve the use of synthetic oligonucleotides suitable as primers for PCR capable of hybridizing to nucleic acids. The sequences of such primers can easily be established by those skilled in the art to be specific to the nucleotide sequences given as Seq ID Nos 11 to 15 for PNMT, Seq ID Nos 21 and 22 for SOD and Seq ID No 25 for paramyosin, respectively. The primers generally can have from 10 to 40 nucleotides, preferably from 15 to 25 nucleotides.

Furthermore, labelled nucleic acid probes capable of hybridizing to mRNA or genomic DNA specific for PNMT, SOD or paramyosin genes are suitable diagnostic tools. The nucleic acid probe can be, for example, a full-length nucleic acid or a portion thereof, such as an oligonucleotide sufficient to specifically hybridize to mRNA or genomic DNA of PNMT, SOD or paramyosin genes, respectively, for example in Northern or Southern hybridizations.

Further the antigens PNMT, SOD and paramyosin, synthetic peptides comprising epitopes specific for these antigens, or antibodies directed against these are useful in the production of diagnostic immunoassays for detecting the presence of *Dictyocaulus viviparus* antigens and/or *anti-Dictyocaulus viviparus* antibodies contained in a biological sample such as serum. Either the *Dictyocaulus viviparus* antigen or antibody specific to the *Dictyocaulus viviparus* antigen is mixed with a sample suspected of containing *Dictyocaulus viviparus* antibody or antigen and monitored for antigen-antibody binding. In a preferred embodiment, the antigen or antibody is immobilized on a solid matrix such that the antigen or antibody is accessible to complementary antibody or antigen contacting a surface of the matrix. The sample then is brought into contact with the surface of the matrix, and the surface is monitored for antigen-antibody binding. Preferably the antigen or antibody will have a detectable label. Antibodies are polyclonal, or more preferably, monoclonal. An intact antibody, or a fragment thereof (e.g., Fab or F(ab')₂) can be used. The term "labelled" is intended to encompass direct labelling by coupling (i.e., physically linking) a detectable substance as well as indirect labelling by reactivity with another reagent that is directly labelled. Indirect labelling is for example the detection of a primary antibody using a fluorescently labelled secondary antibody.

For example, the antigen or antibody can be used in an enzyme-linked immunosorbent assay (ELISA), in which antigen or antibody is bound to a solid phase and an enzyme-antibody or enzyme-antigen conjugate is used to detect and/or quantify antibody or antigen present in a sample. Alternatively, a Western blot assay can be used, in which solubilized and separated antigens are bound to nitrocellulose membrane. The antibody then is detected by an enzyme or label-conjugated anti-immunoglobulin (Ig), such as horseradish peroxidase-Ig conjugate by incubating the membrane in the presence of a precipitable or detectable substrate. Western blot assays have the advantage of not requiring purity greater than 50% for the desired antigen.

The invention also encompasses kits for detecting the presence of *Dictyocaulus viviparus* in a biological sample. For example, the kit can comprise a labelled compound or agent to detect PNMT, SOD or paramyosin in terms of nucleic acids, proteins or antibodies to them, e.g. a diagnostic kit can comprise nucleic acid probes specific for mRNA and/or DNA encoding paramyosin, PNMT and/or SOD, it can comprise monoclonal and/or polyclonal antibody specific for paramyosin, PNMT and/or SOD or it can comprise protein containing epitopes of paramyosin, PNMT and/or SOD (e.g. heterologously expressed full length natural sequences).

## Claims

1. Vaccine suitable for eliciting immunoprotection against Strongylida in ruminants, **characterized by** comprising protein comprising immunoprotective epitopes of at least one protein selected from the group of PNMT (phenyl ethanolamine-N-methyltransferase), paramyosin, and SOD (extra-cellular copper-zinc superoxide dismutase), each derivable from a parasitic nematode of the genus Strongylida.

2. Vaccine according to claim 1, **characterized by** comprising immunoprotective epitopes of PNMT and immunoprotective epitopes at least one protein selected from the group of paramyosin and SOD.

3. Vaccine according to one of the preceding claims, **characterized in that** the parasitic nematode is *Dictyocaulus viviparus.*

4. Vaccine according to one of the preceding claims, **characterized in that** the epitopes for PNMT, SOD and paramyosin are encoded by the *Dictyocaulus viviparus* genome.

5. Vaccine according to one of the preceding claims, **characterized in that** the epitopes are contained in proteins which are essentially identical to the natural translation product of the *Dictyocaulus viviparus* genome.

6. Vaccine according to one of the preceding claims, **characterized in that** PNMT is essentially derivable from one of Seq ID Nos 16 to 20, SOD is essentially derivable from Seq ID Nos 23 or 24, and paramyosin is essentially derivable from Seq ID No 26.

7. Vaccine according to one of the preceding claims, **characterized in that** PNMT is encoded by a nucleic acid sequence hybridizing to one of Seq ID Nos 11 to 15, SOD is encoded by a nucleic acid sequence hybridizing to one of Seq ID Nos 21 and 22, and paramyosin is encoded by a nucleic acid sequence hybridizing to Seq ID No 25.

8. Vaccine according to one of the preceding claims, **characterized by** comprising immunoprotective epitopes derivable from at least one protein selected from the group of MSP (major sperm protein obtainable from *Dictyocaulus* spp.), ACE (acetylcholine esterase obtainable from *Dictyocaulus* spp.), and PDI (protein disulfide isomerase obtainable from *Dictyocaulus* spp.).

9. Vaccine according to one of the preceding claims, **characterized in that** MSP is essentially derivable from Seq ID No 28, ACE is essentially derivable from Seq ID No 30, and PDI is essentially derivable from one of Seq ID Nos 33 and 34.

10. Vaccine according to one of the preceding claims, **characterized in that** MSP is encoded by a nucleic acid sequence hybridizing to Seq ID No 27, ACE is encoded by a nucleic acid sequence hybridizing to Seq ID No 29, and PDI is encoded by a nucleic acid sequence hybridizing to one of Seq ID Nos 31 and 32.

11. Vaccine according to one of the preceding claims, wherein the ruminant is cattle.

12. PNMT suitable for a vaccine according to one of the preceding claims, **characterized in that** it is obtainable from a gene of *Dictyocaulus viviparus.*

13. Paramyosin suitable for a vaccine according to one of claims 1-11, **characterized in that** it is obtainable from a gene *of Dictyocaulus viviparus.*

14. SOD suitable for a vaccine according to one of claims 1-11, **characterized in that** it is obtainable from a gene of *Dictyocaulus viviparus.*

15. Antibody, **characterized by** a specificity against a vaccine according to one of claims 1 - 11.

16. Analytical method for the determination of the presence of a parasitic nematode of the genus Strongylida in an animal, **characterized by** the use of an antibody according to claim 15.

17. Method for passive immunization, **characterized by** the use of antibody according to claim 15.

18. Method for production of a vaccine for ruminants, **characterized by** producing a vaccine according to one of claims 1-11.

19. Method according to claim 18, **characterized by** heterologous expression of at least one of the peptide compounds.


### Annex to the application documents - subsequently filed sequences listing

SEQUENCE LISTING

<110>  Stiftung Tierärztliche Hochschule Hannover

<120>  Vaccine and antibodies against Dictyocaulus viviparus

<130>  N1018EP

<160>  40

<170>  PatentIn version 3.1

<210>  1
<211>  23
<212>  DNA
<213>  artificial sequence

<220>
<223>  primer
<400>  1
acgcgtttag cactactggt tgt                                              23

<210>  2
<211>  18
<212>  DNA
<213>  artificial sequence

<220>
<223>  primer
<400>  2
ggacggaagc tgctacaa                                                    18

<210>  3
<211>  34
<212>  DNA
<213>  artificial sequence

<220>

<223>  primer

<400>  3
cacaaaatcg ccaagagcta atgcggtggt atga                                    34

<210>  4

<211>  35

<212>  DNA

<213>  artificial sequence


<220>

<223>  primer

<400>  4
ggcaaacggt tgtctagcag ctggaggtca ttata                                   35

<210>  5

<211>  32

<212>  DNA

<213>  artificial sequence


<220>

<223>  primer

<400>  5
cttgccgatg cgaatcgaaa actgcatgag tt                                      32

<210>  6

<211>  39

<212>  DNA

<213>  artificial sequence


<220>

<223>  primer

<400>  6
aagctagttt ggaagatacc caacgtcagc tccaacaag                               39

<210>  7

<211>  33

<212>  DNA

<213>  artificial sequence

<220>

<223>  primer

<400>  7
atttcgcgta tcactgtgct gcctcaacac cac                                    33

<210>  8

<211>  35

<212>  DNA

<213>  artificial sequence

<220>

<223>  primer

<400>  8
atacgtatcg cgcccaattc caagcgttta gttta                                  35

<210>  9

<211>  36

<212>  DNA

<213>  artificial sequence

<220>

<223>  primer

<400>  9
agcggtcgat ttcaaattgc atattcttgc gaagag                                 36

<210>  10

<211>  43

<212>  DNA

<213>  artificial sequence

<220>

<223>  primer

<400>  10
gaatagcgat ggtgttctga gctttctcaa ggtcaacaat aag                         43

<210>  11

<211>  1288

<212>  DNA

<213>  Dictyocaulus viviparus

<400>  11
gatcttctac tgagtgatta ttttttccgt ccttttcatt ctactgatac atattttgat      60

cattctgaaa ctctgtggac ttgaaaagga aaaaacgaaa ataagaactc taagcatgga     120

gtcagcagac ggtgaaaatc ttaagcaggc tatcctcgat agagacacat ttcataagga     180

attcaacaca gaagcttatt taaaggactt ttatactaaa gttgaagact cagcaatgca     240

aatggtattg atattcttac ccaatattgt ggctagaatc ggtaaaatta aacgggtttt     300

ggattttgga gcaggaccaa ctattcatgt ggctgcatca tttcgaaatc aagctgtgga     360

gatctatctt gctgattatc taccacaaaa tcgccaagag ctaatgcggt ggtatgaggg     420

aagttcgagt tttgattggt cccatccaat gaaaatgata ttaacgcagg aaggaaatcc     480

atggaatgat ttggacgaaa tgataagaat aacaagacaa aaagttcgtg gtatcttcca     540

ttgtgactgt ttctcatctc catctgttga tgtttccgaa cagctccaag ggatgtttga     600

taccgttgtc acaatctttt gtgtagaata ttgctgcaaa acttatgacg aatacaagaa     660

cgcaattaaa aatattacgc aacaaattcg tgaaggagtc tgtatcagat cgatgtaagc     720

tcagtggtgt tgaggcagca cagtgatacg cgaaatgtag acaacgtcgc gaccacttat     780

acatctaccg aatctgtcat cctttcaggg ttttcttgta atgggaggga tacttgaaga     840

aacatggtgt tcttttggcg gtcgaatgtt ttcatgcctc tacattacta aggaaatgat     900

gcttaatgct atcgaagaag ctggacttcg tatagaaaac gattgcaaat gtattatgta     960

caatatagat aacatgttta tgctttgtgc acgtaaagct aatacgatat ccgataatac    1020

ttcctaattc tactctgttg ttaattattc tacagcttat aataagtgtt tttttttcaa    1080

atgttttttt ttgttttgta aggattcact tgacgaatta tcggtcatat tgctggcttt    1140

tgctttattg ctattaaata ttaaagtagt aatattgtac gaaacatagt tgtaaatctg    1200

ataacatcca aaattcattg aactaaacct aaccattttg ttgacatttc gaagagaaat    1260

aaaattttga atactcaaaa aaaaaaaa                                       1288

<210>  12

<211>  1177

<212>  DNA

<213>  Dictyocaulus viviparus

<400>  12
gatcttctac tgagtgatta ttttttccgt ccttttcatt ctactgatac atattttgat      60

cattctgaaa ctctgtggac ttgaaaagga aaaaacgaaa ataagaactc taagcatgga     120

gtcagcagac ggtgaaaatc ttaagcaggc tatcctcgat agagacacat ttcataagga     180

attcaacaca gaagcttatt taaaggactt ttatactaaa gttgaagact cagcaatgca 240

aatggtattg atattcttac ccaatattgt ggctagaatc ggtaaaatta aacgggtttt 300

ggattttgga gcaggaccaa ctattcatgt ggctgcatca tttcgaaatc aagctgtgga 360

gatctatctt gctgattatc taccacaaaa tcgccaagag ctaatgcggt ggtatgaggg 420

aagttcgagt tttgattggt cccatccaat gaaaatgata ttaacgcagg aaggaaatcc 480

atggaatgat ttggacgaaa tgataagaat aacaagacaa aaagttcgtg gtatcttcca 540

ttgtgactgt ttctcatctc catctgttga tgtttccgaa cagctccaag ggatgtttga 600

taccgttgtc acaatctttt gtgtagaata ttgctgcaaa acttatgacg aatacaagaa 660

cgcaattaaa aatattacgc aacaaattcg tgaaggaggt tttcttgtaa tgggagggat 720

acttgaagaa acatggtgtt cttttggcgg tcgaatgttt tcatgcctct acattactaa 780

ggaaatgatg cttaatgcta tcgaagaagc tggacttcgt atagaaaacg attgcaaatg 840

tattatgtac aatatagata acatgtttat gctttgtgca cgtaaagcta atacgatatc 900

cgataatact tcctaattct actctgttgt taattattct acagcttata ataagtgttt 960

ttttttcaaa tgtttttttt tgttttgtaa ggattcactt gacgaattat cggtcatatt 1020

gctggctttt gctttattgc tattaaatat taaagtagta atattgtacg aaacatagtt 1080

gtaaatctga taacatccaa aattcattga actaaaccta accattttgt tgacatttcg 1140

aagagaaata aaattttgaa tactcaaaaa aaaaaaa 1177

<210> 13

<211> 1382

<212> DNA

<213> Dictyocaulus viviparus

<400> 13
gatcttctac tgagtgatta ttttttccgt ccttttcatt ctactgatac atattttgat 60

cattctgaaa ctctgtggac ttgaaaagga aaaaacgaaa ataagaactc taagcatgga 120

gtcagcagac ggtgaaaatc ttaagcaggc tatcctcgat agagacacat ttcataagga 180

attcaacaca gaagcttatt taaaggactt ttatactaaa gttgaagact cagcaatgca 240

aatggtattg atattcttac ccaatattgt ggctagaatc ggtaaaatta aacgggtttt 300

ggattttgga gcaggaccaa ctattcatgt ggctgcatca tttcgaaatc aagctgtgga 360

gatctatctt gctgattatc taccacaaaa tcgccaagag ctaatgcggt ggtatgaggg 420

aagttcgagt tttgattggt cccatccaat gaaaatgata ttaacgcagg aaggaaatcc 480

atggaatgat ttggacgaaa tgataagaat aacaagacaa aaagttcgtg gtatcttcca 540

ttgtgactgt ttctcatctc catctgttga tgtttccgaa cagctccaag ggatgtttga 600

taccgttgtc acaatctttt gtgtagaata ttgctgcaaa acttatgacg aatacaagaa 660

cgcaattaaa aatattacgc aacaaattcg tgaaggaggt actttttgaa gttattcaag 720
aattcagaat tcaaacacca ctacattcga agtgttcagt ttggtaagat tagtttcagt 780
ttggttttct agtctgtatc agatcgatgt aagctcagtg gtgttgaggc agcacagtga 840
tacgcgaaat gtagacaacg tcgcgaccac ttatacatct accgaatctg tcatcctttc 900
agggttttct tgtaatggga gggatacttg aagaaacatg gtgttctttt ggcggtcgaa 960
tgttttcatg cctctacatt actaaggaaa tgatgcttaa tgctatcgaa gaagctggac 1020
ttcgtataga aaacgattgc aaatgtatta tgtacaatat agataacatg tttatgcttt 1080
gtgcacgtaa agctaatacg atatccgata atacttccta attctactct gttgttaatt 1140
attctacagc ttataataag tgtttttttt tcaaatgttt ttttttgttt tgtaaggatt 1200
cacttgacga attatcggtc atattgctgg cttttgcttt attgctatta aatattaaag 1260
tagtaatatt gtacgaaaca tagttgtaaa tctgataaca tccaaaattc attgaactaa 1320
acctaaccat tttgttgaca tttcgaagag aaataaaatt ttgaatactc aaaaaaaaaa 1380
aa 1382

<210> 14
<211> 1382
<212> DNA
<213> Dictyocaulus viviparus

<400> 14
gatcttctac tgagtgatta ttttttccgt ccttttcatt ctactgatac atattttgat 60
cattctgaaa ctctgtggac ttgaaaagga aaaaacgaaa ataagaactc taagcatgga 120
gtcagcagac ggtgaaaatc ttaagcaggc tatcctcgat agagacacat ttcataagga 180
attcaacaca gaagcttatt taaaggactt ttatactaaa gttgaagact cagcaatgca 240
aatggtattg atattcttac ccaatattgt ggctagaatc ggtaaaatta aacgggtttt 300
ggattttgga gcaggaccaa ctattcatgt ggctgcatca tttcgaaatc aagctgtgga 360
gatctatctt gctgattatc taccacaaaa tcgccaagag ctaatgcggt ggtatgaggg 420
aagttcgagt tttgattggt cccatccaat gaaaatgata ttaacgcagg aaggaaatcc 480
atggaatgat ttggacgaaa tgataagaat aacaagacaa aaagttcgtg gtatcttcca 540
ttgtgactgt ttctcatctc catctgttga tgtttccgaa cagctccaag ggatgtttga 600
taccgttgtc acaatctttt gtgtagaata ttgctgcaaa acttatgacg aatacaagaa 660
cgcaattaaa aatattacgc aacaaattcg tgaaggaggt actttttgaa gttattcaag 720
aattcagaat tcaaacacca ctacattcga agtgttcagt ttggtaagat tagtttcagt 780
ttggttttct agtctgtatc agatcgatgt aagctcagtg gtgttgaggc agcacagtga 840
tacgcgaaat gtagacaacg tcgcgaccac ttatacatct accgaatctg tcatcctttc 900
agggttttct tgtaatggga gggatacttg aagaaacatg gtgttctttt ggcggtcgaa 960

tgttttcatg cctctacatt actaaggaaa tgatgcttaa tgctatcgaa gaagctggac 1020
ttcgtataga aaacgattgc aaatgtatta tgtacaatat agataacatg tttatgcttt 1080
gtgcacgtaa agctaatacg atatccgata atacttccta attctactct gttgttaatt 1140
attctacagc ttataataag tgtttttttt tcaaatgttt ttttttgttt tgtaaggatt 1200
cacttgacga attatcggtc atattgctgg cttttgcttt attgctatta aatattaaag 1260
tagtaatatt gtacgaaaca tagttgtaaa tctgataaca tccaaaattc attgaactaa 1320
acctaaccat tttgttgaca tttcgaagag aaataaaatt ttgaatactc aaaaaaaaaa 1380
aa 1382

<210> 15
<211> 1278
<212> DNA
<213> Dictyocaulus viviparus

<400> 15
gatcttctac tgagtgatta ttttttccgt ccttttcatt ctactgatac atattttgat 60
cattctgaaa ctctgtggac ttgaaaagga aaaaacgaaa ataagaactc taagcatgga 120
gtcagcagac ggtgaaaatc ttaagcaggc tatcctcgat agagacacat ttcataagga 180
attcaacaca gaagcttatt taaaggactt ttatactaaa gttgaagact cagcaatgca 240
aatggtattg atattcttac ccaatattgt ggctagaatc ggtaaaatta aacgggtttt 300
ggattttgga gcaggaccaa ctattcatgt ggctgcatca tttcgaaatc aagctgtgga 360
gatctatctt gctgattatc taccacaaaa tcgccaagag ctaatgcggt ggtatgaggg 420
aagttcgagt tttgattggt cccatccaat gaaaatgata ttaacgcagg aaggaaatcc 480
atggaatgat ttggacgaaa tgataagaat aacaagacaa aaagttcgtg gtatcttcca 540
ttgtgactgt ttctcatctc catctgttga tgtttccgaa cagctccaag ggatgtttga 600
taccgttgtc acaatctttt gtgtagaata ttgctgcaaa acttatgacg aatacaagaa 660
cgcaattaaa aatattacgc aacaaattcg tgaaggagat cgatgtaagc tcagtggtgt 720
tgaggcagca cagtgatacg cgaaatgtag acaacgtcgc gaccacttat acatctaccg 780
aatctgtcat cctttcaggg ttttcttgta atgggaggga tacttgaaga aacatggtgt 840
tcttttggcg gtcgaatgtt ttcatgcctc tacattacta aggaaatgat gcttaatgct 900
atcgaagaag ctggacttcg tatagaaaac gattgcaaat gtattatgta caatatagat 960
aacatgttta tgctttgtgc acgtaaagct aatacgatat ccgataatac ttcctaattc 1020
tactctgttg ttaattattc tacagcttat aataagtgtt tttttttcaa atgttttttt 1080
ttgttttgta aggattcact tgacgaatta tcggtcatat tgctggcttt tgctttattg 1140
ctattaaata ttaaagtagt aatattgtac gaaacatagt tgtaaatctg ataacatcca 1200

aaattcattg aactaaacct aaccattttg ttgacatttc gaagagaaat aaaatttgga    1260

atactcaaaa aaaaaaaa                                                  1278

<210>  16

<211>  200

<212>  PRT

<213>  Dictyocaulus viviparus

<400>  16

Met Glu Ser Ala Asp Gly Glu Asn Leu Lys Gln Ala Ile Leu Asp Arg
1               5                   10                  15

Asp Thr Phe His Lys Glu Phe Asn Thr Glu Ala Tyr Leu Lys Asp Phe
            20                  25                  30

Tyr Thr Lys Val Glu Asp Ser Ala Met Gln Met Val Leu Ile Phe Leu
        35                  40                  45

Pro Asn Ile Val Ala Arg Ile Gly Lys Ile Lys Arg Val Leu Asp Phe
    50                  55                  60

Gly Ala Gly Pro Thr Ile His Val Ala Ala Ser Phe Arg Asn Gln Ala
65                  70                  75                  80

Val Glu Ile Tyr Leu Ala Asp Tyr Leu Pro Gln Asn Arg Gln Glu Leu
                85                  90                  95

Met Arg Trp Tyr Glu Gly Ser Ser Ser Phe Asp Trp Ser His Pro Met
            100                 105                 110

Lys Met Ile Leu Thr Gln Glu Gly Asn Pro Trp Asn Asp Leu Asp Glu
        115                 120                 125

Met Ile Arg Ile Thr Arg Gln Lys Val Arg Gly Ile Phe His Cys Asp
    130                 135                 140

Cys Phe Ser Ser Pro Ser Val Asp Val Ser Glu Gln Leu Gln Gly Met
145                 150                 155                 160

Phe Asp Thr Val Val Thr Ile Phe Cys Val Glu Tyr Cys Cys Lys Thr
                165                 170                 175

Tyr Asp Glu Tyr Lys Asn Ala Ile Lys Asn Ile Thr Gln Gln Ile Arg
            180                 185                 190

Glu Gly Val Cys Ile Arg Ser Met
        195                 200

<210>  17

<211>  266

<212>  PRT

<213>  Dictyocaulus viviparus


<400>  17

Met Glu Ser Ala Asp Gly Glu Asn Leu Lys Gln Ala Ile Leu Asp Arg
1               5                   10                  15

Asp Thr Phe His Lys Glu Phe Asn Thr Glu Ala Tyr Leu Lys Asp Phe
            20                  25                  30

Tyr Thr Lys Val Glu Asp Ser Ala Met Gln Met Val Leu Ile Phe Leu
        35                  40                  45

Pro Asn Ile Val Ala Arg Ile Gly Lys Ile Lys Arg Val Leu Asp Phe
    50                  55                  60

Gly Ala Gly Pro Thr Ile His Val Ala Ala Ser Phe Arg Asn Gln Ala
65                  70                  75                  80

Val Glu Ile Tyr Leu Ala Asp Tyr Leu Pro Gln Asn Arg Gln Glu Leu
                85                  90                  95

Met Arg Trp Tyr Glu Gly Ser Ser Ser Phe Asp Trp Ser His Pro Met
            100                 105                 110

Lys Met Ile Leu Thr Gln Glu Gly Asn Pro Trp Asn Asp Leu Asp Glu
        115                 120                 125

Met Ile Arg Ile Thr Arg Gln Lys Val Arg Gly Ile Phe His Cys Asp
    130                 135                 140

Cys Phe Ser Ser Pro Ser Val Asp Val Ser Glu Gln Leu Gln Gly Met
145                 150                 155                 160

Phe Asp Thr Val Val Thr Ile Phe Cys Val Glu Tyr Cys Cys Lys Thr
                165                 170                 175

Tyr Asp Glu Tyr Lys Asn Ala Ile Lys Asn Ile Thr Gln Gln Ile Arg
            180                 185                 190

Glu Gly Gly Phe Leu Val Met Gly Gly Ile Leu Glu Glu Thr Trp Cys
        195                 200                 205

Ser Phe Gly Gly Arg Met Phe Ser Cys Leu Tyr Ile Thr Lys Glu Met
    210                 215                 220

Met Leu Asn Ala Ile Glu Glu Ala Gly Leu Arg Ile Glu Asn Asp Cys

225                 230                 235                 240

Lys Cys Ile Met Tyr Asn Ile Asp Asn Met Phe Met Leu Cys Ala Arg
                245                 250                 255

Lys Ala Asn Thr Ile Ser Asp Asn Thr Ser
            260                 265

<210>  18

<211>  178

<212>  PRT

<213>  Dictyocaulus viviparus

<400>  18

Met Glu Ser Ala Asp Gly Glu Asn Leu Lys Gln Ala Ile Leu Asp Arg
1               5                   10                  15

Asp Thr Phe His Lys Glu Phe Asn Thr Glu Ala Tyr Leu Lys Asp Phe
            20                  25                  30

Tyr Thr Lys Val Glu Asp Ser Ala Met Gln Met Val Leu Ile Phe Leu
        35                  40                  45

Pro Asn Ile Val Ala Arg Ile Gly Lys Ile Lys Arg Val Leu Asp Phe
    50                  55                  60

Gly Ala Gly Pro Thr Ile His Val Ala Ala Ser Phe Arg Asn Gln Ala
65                  70                  75                  80

Val Glu Ile Tyr Leu Ala Asp Tyr Leu Pro Gln Asn Arg Gln Glu Leu
                85                  90                  95

Met Arg Trp Tyr Glu Gly Ser Ser Ser Phe Asp Trp Ser His Pro Met
            100                 105                 110

Lys Met Ile Leu Thr Gln Glu Gly Asn Pro Trp Asn Asp Leu Asp Glu
        115                 120                 125

Met Ile Arg Ile Thr Arg Gln Lys Val Arg Gly Ile Phe His Cys Asp
    130                 135                 140

Cys Phe Ser Ser Pro Ser Val Asp Val Ser Glu Gln Leu Gln Gly Met
145                 150                 155                 160

Phe Asp Val Lys Phe Arg Ile Ser Phe Phe Phe Asn Asp Val Lys Lys
                165                 170                 175

Leu Tyr

<210>  19

<211>  197

<212>  PRT

<213>  Dictyocaulus viviparus

<400>  19

Met Glu Ser Ala Asp Gly Glu Asn Leu Lys Gln Ala Ile Leu Asp Arg
1               5                   10                  15

Asp Thr Phe His Lys Glu Phe Asn Thr Glu Ala Tyr Leu Lys Asp Phe
            20                  25                  30

Tyr Thr Lys Val Glu Asp Ser Ala Met Gln Met Val Leu Ile Phe Leu
        35                  40                  45

Pro Asn Ile Val Ala Arg Ile Gly Lys Ile Lys Arg Val Leu Asp Phe
    50                  55                  60

Gly Ala Gly Pro Thr Ile His Val Ala Ala Ser Phe Arg Asn Gln Ala
65                  70                  75                  80

Val Glu Ile Tyr Leu Ala Asp Tyr Leu Pro Gln Asn Arg Gln Glu Leu
                85                  90                  95

Met Arg Trp Tyr Glu Gly Ser Ser Ser Phe Asp Trp Ser His Pro Met
            100                 105                 110

Lys Met Ile Leu Thr Gln Glu Gly Asn Pro Trp Asn Asp Leu Asp Glu
        115                 120                 125

Met Ile Arg Ile Thr Arg Gln Lys Val Arg Gly Ile Phe His Cys Asp
    130                 135                 140

Cys Phe Ser Ser Pro Ser Val Asp Val Ser Glu Gln Leu Gln Gly Met
145                 150                 155                 160

Phe Asp Thr Val Val Thr Ile Phe Cys Val Glu Tyr Cys Cys Lys Thr
                165                 170                 175

Tyr Asp Glu Tyr Lys Asn Ala Ile Lys Asn Ile Thr Gln Gln Ile Arg
            180                 185                 190

Glu Gly Gly Thr Phe
        195

<210>  20

<211>  206

<212>  PRT

<213>  Dictyocaulus viviparus

<400>  20

Met Glu Ser Ala Asp Gly Glu Asn Leu Lys Gln Ala Ile Leu Asp Arg
1               5                   10                  15

Asp Thr Phe His Lys Glu Phe Asn Thr Glu Ala Tyr Leu Lys Asp Phe
            20                  25                  30

Tyr Thr Lys Val Glu Asp Ser Ala Met Gln Met Val Leu Ile Phe Leu
        35                  40                  45

Pro Asn Ile Val Ala Arg Ile Gly Lys Ile Lys Arg Val Leu Asp Phe
    50                  55                  60

Gly Ala Gly Pro Thr Ile His Val Ala Ala Ser Phe Arg Asn Gln Ala
65                  70                  75                  80

Val Glu Ile Tyr Leu Ala Asp Tyr Leu Pro Gln Asn Arg Gln Glu Leu
                85                  90                  95

Met Arg Trp Tyr Glu Gly Ser Ser Ser Phe Asp Trp Ser His Pro Met
            100                 105                 110

Lys Met Ile Leu Thr Gln Glu Gly Asn Pro Trp Asn Asp Leu Asp Glu
        115                 120                 125

Met Ile Arg Ile Thr Arg Gln Lys Val Arg Gly Ile Phe His Cys Asp
    130                 135                 140

Cys Phe Ser Ser Pro Ser Val Asp Val Ser Glu Gln Leu Gln Gly Met
145                 150                 155                 160

Phe Asp Thr Val Val Thr Ile Phe Cys Val Glu Tyr Cys Cys Lys Thr
                165                 170                 175

Tyr Asp Glu Tyr Lys Asn Ala Ile Lys Asn Ile Thr Gln Gln Ile Arg
            180                 185                 190

Glu Gly Asp Arg Cys Lys Leu Ser Gly Val Glu Ala Ala Gln
        195                 200                 205

<210>  21

<211>  1035

<212>  DNA

<213>  Dictyocaulus viviparus

<400>  21
acacacatca tgatactcca catttcactc atcatatcga cgattctctt aggtgttcac      60

gcacatggta atctatgcag aaatggagct tttatgaacg ttgtaaaagc tcgtgcgtat     120

atgtttgaag ctgtaccgga cggtgatcca caaaaattga ttggaattat tgattttgtg     180

caatacagat ctttagtgaa gcttaacggt actgtatctg gattgaaatc aggacttcat     240

ggatttcatg tacatgaaaa aggtaatttg gcaaacggtt gtctagcagc tggaggtcat     300

tataatcctt acaaattgat gcacggtgca ccaagtgact ctaatcgaca tgtcggtgat     360

ctgggaagca tcgttacctc agctaacggc gaaactgtga tttcaatttc tgacccagtg     420

attacactca atggttatca cagtgtgatt ggacgagccg ttgtcattca tgccgatgca     480

gatgacttag gccttggcag atcggagatg agcaagtcaa ctggcaattc tggagctcga     540

gtcgcgtgtg gagtcatcgg tatcgtctaa actaaacgct tggaattggg cgcgatacgt     600

atcccgattt ctgcaatcct atcaaaaaac aaatcattat catattatta ctgtttacag     660

agtaacgtac tagtcacgat agtttttatc gggtgcctat ttcatttaca atatgcattt     720

tgaagtgtat cgtttgtagg cgtttctgaa gaggtgggag atgtgtgcct ctgaagaatc     780

agaatcatca ctatcgtcac aaaatgagga catccataac gaaaagaatt cttctacatg     840

taatttcatt aactatttcc tcactactat tctgctgttt gtattgtcaa cctcactagt     900

aaatataatc taggggaaat ttaagtccca caaaatcatt ttatatcttt aacaatccat     960

ctcagcttca tctcttcgaa taccgctatt cttgcagggt aagttatatt aataaagaaa    1020

tttctttcca aaaaa                                                     1035

<210>  22

<211>  1032

<212>  DNA

<213>  Dictyocaulus viviparus

<400>  22
acacacatca tgatactcca catttcactc atatcgacga ttctcttagg tgttcacgca      60

catggtaatc tatgcagaaa tggagctttt atgaacgttg taaaagctcg tgcgtatatg     120

tttgaagctg taccggacgg tgatccacaa aaattgattg gaattattga ttttgtgcaa     180

tacagatctt tagtgaagct taacggtact gtatctggat tgaaatcagg acttcatgga     240

tttcatgtac atgaaaaagg taatttggca aacggttgtc tagcagctgg aggtcattat     300

aatccttaca aattgatgca cggtgcacca agtgactcta atcgacatgt cggtgatctg     360

ggaaacatcg ttacctcagc taacggcgaa actgtgattt caatttctga cccagtgatt     420

acactcaatg gttatcacag tgtgattgga cgagccgttg tcattcatgc cgatgcagat     480

gacttaggcc ttggcagatc ggaaatgagc aagtcaactg gcaattctgg agctcgagtc     540

gcgtgtggag tcatcggtat cgtctaaact aaacgcttgg aattgggcgc gatacgtatc 600

ccgatttctg caatcctatc aaaaaacaaa tcattatcat attattactg tttacagagt 660

aacgtactag tcacgatagt ttttatcggg tgcctatttc atttacaata tgcattttga 720

agtgtatcgt ttgtaggcgt ttctgaagag gtgggagatg tgtgcctctg aagaatcaga 780

atcatcacta tcgtcacaaa atgaggacat ccataacgaa aagaattctt ctacatgtaa 840

tttcattaac tatttcctca ctactattct gctgtttgta ttgtcaacct cactagtaaa 900

tataatctag gggaaattta agtcccacaa aatcatttta tatctttaac aatccatctc 960

agcttcatct cttcgaatac cgctattctt gcagggtaag ttatattaat aaagaaattt 1020

ctttccaaaa aa 1032

<210> 23

<211> 186

<212> PRT

<213> Dictyocaulus viviparus

<400> 23

Met Ile Leu His Ile Ser Leu Ile Ile Ser Thr Ile Leu Leu Gly Val
1               5                   10                  15

His Ala His Gly Asn Leu Cys Arg Asn Gly Ala Phe Met Asn Val Val
            20                  25                  30

Lys Ala Arg Ala Tyr Met Phe Glu Ala Val Pro Asp Gly Asp Pro Gln
        35                  40                  45

Lys Leu Ile Gly Ile Ile Asp Phe Val Gln Tyr Arg Ser Leu Val Lys
    50                  55                  60

Leu Asn Gly Thr Val Ser Gly Leu Lys Ser Gly Leu His Gly Phe His
65                  70                  75                  80

Val His Glu Lys Gly Asn Leu Ala Asn Gly Cys Leu Ala Ala Gly Gly
                85                  90                  95

His Tyr Asn Pro Tyr Lys Leu Met His Gly Ala Pro Ser Asp Ser Asn
            100                 105                 110

Arg His Val Gly Asp Leu Gly Ser Ile Val Thr Ser Ala Asn Gly Glu
        115                 120                 125

Thr Val Ile Ser Ile Ser Asp Pro Val Ile Thr Leu Asn Gly Tyr His
    130                 135                 140

Ser Val Ile Gly Arg Ala Val Val Ile His Ala Asp Ala Asp Asp Leu
145                 150                 155                 160

Gly Leu Gly Arg Ser Glu Met Ser Lys Ser Thr Gly Asn Ser Gly Ala
                165                 170                 175

Arg Val Ala Cys Gly Val Ile Gly Ile Val
            180                 185

<210>  24

<211>  185

<212>  PRT

<213>  Dictyocaulus viviparus

<400>  24

Met Ile Leu His Ile Ser Leu Ile Ser Thr Ile Leu Leu Gly Val His
1               5                   10                  15

Ala His Gly Asn Leu Cys Arg Asn Gly Ala Phe Met Asn Val Val Lys
            20                  25                  30

Ala Arg Ala Tyr Met Phe Glu Ala Val Pro Asp Gly Asp Pro Gln Lys
        35                  40                  45

Leu Ile Gly Ile Ile Asp Phe Val Gln Tyr Arg Ser Leu Val Lys Leu
    50                  55                  60

Asn Gly Thr Val Ser Gly Leu Lys Ser Gly Leu His Gly Phe His Val
65                  70                  75                  80

His Glu Lys Gly Asn Leu Ala Asn Gly Cys Leu Ala Ala Gly Gly His
                85                  90                  95

Tyr Asn Pro Tyr Lys Leu Met His Gly Ala Pro Ser Asp Ser Asn Arg
            100                 105                 110

His Val Gly Asp Leu Gly Asn Ile Val Thr Ser Ala Asn Gly Glu Thr
        115                 120                 125

Val Ile Ser Ile Ser Asp Pro Val Ile Thr Leu Asn Gly Tyr His Ser
    130                 135                 140

Val Ile Gly Arg Ala Val Val Ile His Ala Asp Ala Asp Asp Leu Gly
145                 150                 155                 160

Leu Gly Arg Ser Glu Met Ser Lys Ser Thr Gly Asn Ser Gly Ala Arg
                165                 170                 175

Val Ala Cys Gly Val Ile Gly Ile Val
            180                 185

<210> 25

<211> 3215

<212> DNA

<213> Dictyocaulus viviparus

<400> 25
gagtcgacct gggagtttag tgtgcgctct agtccattaa tctaccatca gtctacacat 60

ccttcgtcag ctatcttcag ttagtcatgt ctgctaactt gtacagatcg ccgtcacagg 120

ctgctgtcta caagtctcca tcgatgagtg cctttggtgc accgttcggc agcatgtctg 180

ttgccgatct tggatcgctc acccgtctcg aggataaaat tcgacttctc caggaagacc 240

tcgagtccga acgagagctc agaaaccgaa tcgaacgcga acgcgcagat ctaagtgtgc 300

agcttatcgc tcttactgat cgtctcgaag atgctgaagg tacaaccgac agccagatcg 360

aatcgaatcg taaacgtgag ggagaactca ttaagctgcg caagttgctc gaagaatcac 420

aacttgaatc ggaagatgct atgaacgtac ttcgtaaaaa acaccaagat gcatgcctcg 480

attatcagga tcaagttgaa cagctacaga agaagaacgc taagattgac cgtgaacgtc 540

agcgtctaca gcatgaagtt attgagctaa cagccaccat cgaccaagtg cagaaagata 600

agcacatcgc tgaaaaagct gctgaacgat ttgaagctca aagtattgaa ctgtcaaata 660

aggttgagga tcttaaccga catgtgaatg atctagctca acagcgtcaa cgtctgcaag 720

ctgagaataa cgatcttcta aaagaagtcc atgatcagaa agtacagctc gataatctgc 780

agcacgtcaa atacactctt gctcaacaac ttgaggaagc aagacgccgt ctggaggacg 840

ccgaaaggga acgatcacaa cttcagtctc agcttcatca agttcaactt gagcttgact 900

ctgtacgtac tgcacttgat gaggaatctg cagctcgctc tgatgctgag cacaaactga 960

atcttgcaaa cactgaaatt actcaatgga agagcaaatt cgacgctgag gtcgctctcc 1020

atcatgaaga agtggaagat cttcgtaaaa agatgctgca gaagcaggct gaatatgaag 1080

agcagattga gattatgttg caaaagatta gtcagcttga gaaggcaaag agccgtctgc 1140

aatccgaagt ggaagtactt attgttgacc ttgagaaagc tcagaacacc atcgctattc 1200

ttgaaagagc taaggaacag cttgagaagc aatgtgcaga actcaaggtt cgcatcgatg 1260

aattgaatgt tgaactagag gctgctcagc gcgagctacg cgctgtaaat gctgagttgc 1320

aaaagatgaa acacctatac gagaaggctg ttgaacaaaa ggaggctcta gctagagaaa 1380

ataagaagct tcacgatgag cttcatgagg ctaaagaagc tcttgccgat gcgaatcgaa 1440

aactgcatga gttggatctg gaaaatgctc gtctggctgg agaaattcgc gagttgcaga 1500

cggctcttaa ggaggctgat gctcaacgtc gtgatgccga aaaccgcgcg caacgtgctc 1560

tcgctgaact tcaggctttg cgcattgaaa tggaacgaag acttcaagag aaagaagagg 1620

gaatggaagc tcttcgcaag aatatgcaat ttgaaatcga ccgcttgact gctgcgttgg 1680

ccgatgcaga ggcacgcatg aaggcagaaa tttctcgact gaagaaaaaa taccaagctg 1740

aaattgccga gctggaaatg actgtcgaca atttgaatcg tgctaacatt gaggcgcaaa 1800

agacaatcaa gaaacagtcc gggcaactca aaatactgca agctagtttg gaagataccc 1860

aacgtcagct ccaacaagta ctcgaccaat acgctttagc tcagcgaaag gtcgctgctc 1920

tatcagccga acttgaagag gctaagaccg cccttgataa tgccattcgt gcccgcaaac 1980

aggctgaaat cgatcttgag gaagctaatg gacgaattgc cgacctgtct gcgatcaaca 2040

acaatctcac ttccatcaag aacaaactcg aaaccgaact ttcaaccgct caggccgacc 2100

tcgatgaggt tactaaagaa ctgcacgccg ctgatgagcg cgctaatcgt gctcttgctg 2160

atgcagcccg tgctgtcgaa caattacatg aggaacagga acattctatg aagatcgatg 2220

cacttcgcaa gtctctcgaa gaacaagtaa aacaacttca agttcaaatt caagaagcag 2280

aagcagctgc attactgggt ggaaagcgtg ttattgctaa gcttgaaact cgcatccgag 2340

atcttgaaac tgcccttgat gaagagacgc gcagacacaa agaaacccaa aatgctcttc 2400

gaaagaagga tcgccgcatc aaggaagttc agcagttggt ggatgaagag cacaagaact 2460

tcgttatggc tcaagacaca gccgatcgct tgcaggagaa attgaacatt caaaagagac 2520

aactcgctga agctgaatca gttacgatgg caaatctaca gagagttagg cgatatcaac 2580

acgagcttga agacgcggaa ggtcgcgcag atcaggctga atccagtcta catcttatcc 2640

gcgctaagca tcgttcttct gttgttactg gaaagaccgg cgctagcaaa gtttacgtga 2700

tggaagacga tttctaaggt gaccgcaaaa ttactgctgc tcagccagtg tcgtgaatta 2760

gctacgagcg gccttgtatt tggtattaag tattgttata tgttgtagtg cagtattgct 2820

tgagagcgtg acttttgatt cctatcaaac gaagtcgttt tgctctttct tctttttttg 2880

cgcatggctt cttttcgatc gactttggtt tatcggcgaa tctgtgtcgt aaattggtgc 2940

cttaattcat ttatttctca tttcatttgt atgattagga aaagatatag aatcgcaatc 3000

atttttctta ccggcatgtt ttatttatag tcggttttat tatcatcgct atcgcatact 3060

caattggatg tcgcgtaagc acacatattt atggaaacag tggtattata tttttttgga 3120

aaatgcccta accttttagc tgcaattctt cctttctcac tctctgcctt atgaagtgtt 3180

ggacgagtaa ataaaagtta tttgaacaaa aaaaa 3215

<210> 26

<211> 876

<212> PRT

<213> Dictyocaulus viviparus

<400> 26

Met Ser Ala Asn Leu Tyr Arg Ser Pro Ser Gln Ala Ala Val Tyr Lys
1               5                   10                  15

Ser Pro Ser Met Ser Ala Phe Gly Ala Pro Phe Gly Ser Met Ser Val

20                          25                          30

Ala Asp Leu Gly Ser Leu Thr Arg Leu Glu Asp Lys Ile Arg Leu Leu
        35                  40                  45

Gln Glu Asp Leu Glu Ser Glu Arg Glu Leu Arg Asn Arg Ile Glu Arg
    50                  55                  60

Glu Arg Ala Asp Leu Ser Val Gln Leu Ile Ala Leu Thr Asp Arg Leu
65                  70                  75                  80

Glu Asp Ala Glu Gly Thr Thr Asp Ser Gln Ile Glu Ser Asn Arg Lys
                85                  90                  95

Arg Glu Gly Glu Leu Ile Lys Leu Arg Lys Leu Leu Glu Glu Ser Gln
            100                 105                 110

Leu Glu Ser Glu Asp Ala Met Asn Val Leu Arg Lys Lys His Gln Asp
        115                 120                 125

Ala Cys Leu Asp Tyr Gln Asp Gln Val Glu Gln Leu Gln Lys Lys Asn
    130                 135                 140

Ala Lys Ile Asp Arg Glu Arg Gln Arg Leu Gln His Glu Val Ile Glu
145                 150                 155                 160

Leu Thr Ala Thr Ile Asp Gln Val Gln Lys Asp Lys His Ile Ala Glu
                165                 170                 175

Lys Ala Ala Glu Arg Phe Glu Ala Gln Ser Ile Glu Leu Ser Asn Lys
            180                 185                 190

Val Glu Asp Leu Asn Arg His Val Asn Asp Leu Ala Gln Gln Arg Gln
        195                 200                 205

Arg Leu Gln Ala Glu Asn Asn Asp Leu Leu Lys Glu Val His Asp Gln
    210                 215                 220

Lys Val Gln Leu Asp Asn Leu Gln His Val Lys Tyr Thr Leu Ala Gln
225                 230                 235                 240

Gln Leu Glu Glu Ala Arg Arg Arg Leu Glu Asp Ala Glu Arg Glu Arg
                245                 250                 255

Ser Gln Leu Gln Ser Gln Leu His Gln Val Gln Leu Glu Leu Asp Ser
            260                 265                 270

Val Arg Thr Ala Leu Asp Glu Glu Ser Ala Ala Arg Ser Asp Ala Glu
        275                 280                 285

His Lys Leu Asn Leu Ala Asn Thr Glu Ile Thr Gln Trp Lys Ser Lys
    290                 295                 300

Phe Asp Ala Glu Val Ala Leu His His Glu Glu Val Glu Asp Leu Arg
305                 310                 315                 320

Lys Lys Met Leu Gln Lys Gln Ala Glu Tyr Glu Glu Gln Ile Glu Ile
                325                 330                 335

Met Leu Gln Lys Ile Ser Gln Leu Glu Lys Ala Lys Ser Arg Leu Gln
            340                 345                 350

Ser Glu Val Glu Val Leu Ile Val Asp Leu Glu Lys Ala Gln Asn Thr
        355                 360                 365

Ile Ala Ile Leu Glu Arg Ala Lys Glu Gln Leu Glu Lys Gln Cys Ala
    370                 375                 380

Glu Leu Lys Val Arg Ile Asp Glu Leu Asn Val Glu Leu Glu Ala Ala
385                 390                 395                 400

Gln Arg Glu Leu Arg Ala Val Asn Ala Glu Leu Gln Lys Met Lys His
                405                 410                 415

Leu Tyr Glu Lys Ala Val Glu Gln Lys Glu Ala Leu Ala Arg Glu Asn
            420                 425                 430

Lys Lys Leu His Asp Glu Leu His Glu Ala Lys Glu Ala Leu Ala Asp
        435                 440                 445

Ala Asn Arg Lys Leu His Glu Leu Asp Leu Glu Asn Ala Arg Leu Ala
    450                 455                 460

Gly Glu Ile Arg Glu Leu Gln Thr Ala Leu Lys Glu Ala Asp Ala Gln
465                 470                 475                 480

Arg Arg Asp Ala Glu Asn Arg Ala Gln Arg Ala Leu Ala Glu Leu Gln
                485                 490                 495

Ala Leu Arg Ile Glu Met Glu Arg Arg Leu Gln Glu Lys Glu Glu Gly
            500                 505                 510

Met Glu Ala Leu Arg Lys Asn Met Gln Phe Glu Ile Asp Arg Leu Thr
        515                 520                 525

Ala Ala Leu Ala Asp Ala Glu Ala Arg Met Lys Ala Glu Ile Ser Arg
    530                 535                 540

Leu Lys Lys Lys Tyr Gln Ala Glu Ile Ala Glu Leu Glu Met Thr Val
545                 550                 555                 560

Asp Asn Leu Asn Arg Ala Asn Ile Glu Ala Gln Lys Thr Ile Lys Lys
                565                 570                 575

Gln Ser Gly Gln Leu Lys Ile Leu Gln Ala Ser Leu Glu Asp Thr Gln
            580                 585                 590

Arg Gln Leu Gln Gln Val Leu Asp Gln Tyr Ala Leu Ala Gln Arg Lys
        595                 600                 605

Val Ala Ala Leu Ser Ala Glu Leu Glu Glu Ala Lys Thr Ala Leu Asp
    610                 615                 620

Asn Ala Ile Arg Ala Arg Lys Gln Ala Glu Ile Asp Leu Glu Glu Ala
625                 630                 635                 640

Asn Gly Arg Ile Ala Asp Leu Ser Ala Ile Asn Asn Asn Leu Thr Ser
                645                 650                 655

Ile Lys Asn Lys Leu Glu Thr Glu Leu Ser Thr Ala Gln Ala Asp Leu
            660                 665                 670

Asp Glu Val Thr Lys Glu Leu His Ala Ala Asp Glu Arg Ala Asn Arg
        675                 680                 685

Ala Leu Ala Asp Ala Ala Arg Ala Val Glu Gln Leu His Glu Glu Gln
    690                 695                 700

Glu His Ser Met Lys Ile Asp Ala Leu Arg Lys Ser Leu Glu Glu Gln
705                 710                 715                 720

Val Lys Gln Leu Gln Val Gln Ile Gln Glu Ala Glu Ala Ala Ala Leu
                725                 730                 735

Leu Gly Gly Lys Arg Val Ile Ala Lys Leu Glu Thr Arg Ile Arg Asp
            740                 745                 750

Leu Glu Thr Ala Leu Asp Glu Glu Thr Arg Arg His Lys Glu Thr Gln
        755                 760                 765

Asn Ala Leu Arg Lys Lys Asp Arg Arg Ile Lys Glu Val Gln Gln Leu
    770                 775                 780

Val Asp Glu Glu His Lys Asn Phe Val Met Ala Gln Asp Thr Ala Asp
785                 790                 795                 800

Arg Leu Gln Glu Lys Leu Asn Ile Gln Lys Arg Gln Leu Ala Glu Ala
                805                 810                 815

Glu Ser Val Thr Met Ala Asn Leu Gln Arg Val Arg Arg Tyr Gln His
            820                 825                 830

Glu Leu Glu Asp Ala Glu Gly Arg Ala Asp Gln Ala Glu Ser Ser Leu
        835                 840                 845

His Leu Ile Arg Ala Lys His Arg Ser Ser Val Val Thr Gly Lys Thr
    850                 855                 860

Gly Ala Ser Lys Val Tyr Val Met Glu Asp Asp Phe
865                 870                 875

<210>  27

<211>  472

<212>  DNA

<213>  Dictyocaulus viviparus

<400>  27
aattcgcggc cgcaaacaat ggcgtcagtt ccccctggag acatcaacac tcagccaaac      60

tcgaagatcg tcttcaacgc tccatacgac gacaagcaca cctatcacat caagatcatc     120

aacgcatctg gacgtcgaat tggatgggct atcaagacca ccaacatgaa gagacttggc     180

gtcgacccag cctgcggagt tcttgacccc aaggaggcta ctctcatggc tgtgtcttgc     240

gacaccttcg agtacggacg tgaggacaca aacaacgacc gtatcactgt tgagtggtgc     300

aacacccccg atggcgctgc taagcagttc agacgcgagt ggttccaagg agatggtatg     360

gtccgcagaa agaaccttcc catcgagtac aatccatgaa cacatctggt gcttaataaa     420

taattaagca tggtggttat acactatgct taacaaaatc gaagcggccg cg             472

<210>  28

<211>  126

<212>  PRT

<213>  Dictyocaulus viviparus

<400>  28

Met Ala Ser Val Pro Pro Gly Asp Ile Asn Thr Gln Pro Asn Ser Lys
1               5                   10                  15

Ile Val Phe Asn Ala Pro Tyr Asp Asp Lys His Thr Tyr His Ile Lys
            20                  25                  30

Ile Ile Asn Ala Ser Gly Arg Arg Ile Gly Trp Ala Ile Lys Thr Thr
        35                  40                  45

Asn Met Lys Arg Leu Gly Val Asp Pro Ala Cys Gly Val Leu Asp Pro
    50                  55                  60

Lys Glu Ala Thr Leu Met Ala Val Ser Cys Asp Thr Phe Glu Tyr Gly
65                  70                  75                  80

Arg Glu Asp Thr Asn Asn Asp Arg Ile Thr Val Glu Trp Cys Asn Thr

85                  90                  95

Pro Asp Gly Ala Ala Lys Gln Phe Arg Arg Glu Trp Phe Gln Gly Asp
            100                 105                 110

Gly Met Val Arg Arg Lys Asn Leu Pro Ile Glu Tyr Asn Pro
        115                 120                 125

<210>  29

<211>  1839

<212>  DNA

<213>  Dictyocaulus viviparus

<400>  29
ttgttcacac agacaactgc ttttctcttc tgcactcttc tacatccggc tttcgcgaga      60

gtccactttg aaggaaatga gccttttgtc tggacgcaac ttggtggttt tcgaggagtc     120

taccagactg tcaatgaaaa aagagtggca gcctttctag gagtgccttt cgcacaacct     180

ccaattggag tattacgatt tcagaagcca aaaccagtgg aacattggtg gcatgagtac     240

tgtcctccaa aaaacacggg cctgggtgaa ttagaatgcc atggaaaaga acgagatgaa     300

aacgaatgga aagttccttg caaagggtgc gtactcgacg taattaagcc agcaaactct     360

tgcatgctta cgccagacac aaatttcgga gaatttccag ggtcagagat gtggaatcca     420

ccagagtcat caaaaatcag cgaggattgc ttgtacttga atatgtgggt tcctgagaat     480

cctactggtg atgtgctagt gtggattttc ggtggaggct ttttcagtgg atcaccctca     540

cttgatttat acaatggaag tgtgctagct gctgaacata gtcttatcgt tgtgaacatc     600

aattatcgat tgggaccatt tggtttcctc ttcttcggcc acgaatcacc tgtgcaagga     660

aacatgggtc ttttagacca acagcttgcg ttagagtgga taaataaata tataagggca     720

tttggtggag atcctaagaa ggtgacccta tttggcgagt cagccggatc tgcctccgtt     780

acagcacatc tgctggccag tggtagccat gaatatttta gtaaaataat tggcttaagt     840

ggttcactta caaacttatg ggcgactcga tctccagaaa cattgaagaa agtgtcattt     900

gaatttgcaa agaacttgag ctgcgtcgat atcgaagatc tttcagctgt cttagaatgt     960

ttgaaaacga aatcagcgga ggacgtacag agtgctggct acgcgatgtt cggtctcgaa    1020

cctccaatgt cattcccatt tgtgcctgtt gacgaggata aagtattctt taacggaaat    1080

gttcgcgata taatagagga aggaaaattc aaaaaagttc ccgccgtaat gggtacggtg    1140

aatgacgaag gaacattctg gttaccgtac tacttgaagg agacgggctt cactttcgat    1200

agctcattgc cggcggatgc tgatgagaat aaggcactca taaccgaaga agaatattta    1260

aagtctatgg attcatttgc tccttatttt aaagaagtga ctgaagaaca ttggcaaaat    1320

cttgttgaag catataggaa tgtgtttcct gaagagctac caatcgggga acgacttcga    1380

aatggcgtag gacgattttt aggtgatttg ttcttcacct gcgatttagc agattttacc    1440

aacaagtttg ttaaaaacca agaagattct gtctacagtt attactttaa tatgcgatca 1500

tctgccaatc catggccgga atggatagga gttatgcatg gctatgaaat tgaatacatg 1560

tttggtcaac cgtttttcat gccatcagtc tataaagaag aactgcttga actagaaagg 1620

gaattctcca agtatatgat gaagctgtta gcagatttcg ttcgtgacgg aaaaccttcc 1680

gatgactggt tgccatatac tacaactgat cgtaaagcac tcgttcttga taaagattcc 1740

atggaaggta acacgaaata cgtcgacctt catgaggagc aatgccagtt aatcaaagac 1800

gctcatcctt ctcccggaga accgagtagc acaagcgaa 1839

<210> 30

<211> 613

<212> PRT

<213> Dictyocaulus viviparus

<400> 30

Leu Phe Thr Gln Thr Thr Ala Phe Leu Phe Cys Thr Leu Leu His Pro
1               5                   10                  15

Ala Phe Ala Arg Val His Phe Glu Gly Asn Glu Pro Phe Val Trp Thr
            20                  25                  30

Gln Leu Gly Gly Phe Arg Gly Val Tyr Gln Thr Val Asn Glu Lys Arg
        35                  40                  45

Val Ala Ala Phe Leu Gly Val Pro Phe Ala Gln Pro Pro Ile Gly Val
    50                  55                  60

Leu Arg Phe Gln Lys Pro Lys Pro Val Glu His Trp Trp His Glu Tyr
65                  70                  75                  80

Cys Pro Pro Lys Asn Thr Gly Leu Gly Glu Leu Glu Cys His Gly Lys
                85                  90                  95

Glu Arg Asp Glu Asn Glu Trp Lys Val Pro Cys Lys Gly Cys Val Leu
            100                 105                 110

Asp Val Ile Lys Pro Ala Asn Ser Cys Met Leu Thr Pro Asp Thr Asn
        115                 120                 125

Phe Gly Glu Phe Pro Gly Ser Glu Met Trp Asn Pro Pro Glu Ser Ser
    130                 135                 140

Lys Ile Ser Glu Asp Cys Leu Tyr Leu Asn Met Trp Val Pro Glu Asn
145                 150                 155                 160

Pro Thr Gly Asp Val Leu Val Trp Ile Phe Gly Gly Gly Phe Phe Ser

                165                 170                 175

Gly Ser Pro Ser Leu Asp Leu Tyr Asn Gly Ser Val Leu Ala Ala Glu
            180                 185                 190

His Ser Leu Ile Val Val Asn Ile Asn Tyr Arg Leu Gly Pro Phe Gly
        195                 200                 205

Phe Leu Phe Phe Gly His Glu Ser Pro Val Gln Gly Asn Met Gly Leu
    210                 215                 220

Leu Asp Gln Gln Leu Ala Leu Glu Trp Ile Asn Lys Tyr Ile Arg Ala
225                 230                 235                 240

Phe Gly Gly Asp Pro Lys Lys Val Thr Leu Phe Gly Glu Ser Ala Gly
                245                 250                 255

Ser Ala Ser Val Thr Ala His Leu Leu Ala Ser Gly Ser His Glu Tyr
            260                 265                 270

Phe Ser Lys Ile Ile Gly Leu Ser Gly Ser Leu Thr Asn Leu Trp Ala
        275                 280                 285

Thr Arg Ser Pro Glu Thr Leu Lys Lys Val Ser Phe Glu Phe Ala Lys
    290                 295                 300

Asn Leu Ser Cys Val Asp Ile Glu Asp Leu Ser Ala Val Leu Glu Cys
305                 310                 315                 320

Leu Lys Thr Lys Ser Ala Glu Asp Val Gln Ser Ala Gly Tyr Ala Met
                325                 330                 335

Phe Gly Leu Glu Pro Pro Met Ser Phe Pro Phe Val Pro Val Asp Glu
            340                 345                 350

Asp Lys Val Phe Phe Asn Gly Asn Val Arg Asp Ile Ile Glu Glu Gly
        355                 360                 365

Lys Phe Lys Lys Val Pro Ala Val Met Gly Thr Val Asn Asp Glu Gly
    370                 375                 380

Thr Phe Trp Leu Pro Tyr Tyr Leu Lys Glu Thr Gly Phe Thr Phe Asp
385                 390                 395                 400

Ser Ser Leu Pro Ala Asp Ala Asp Glu Asn Lys Ala Leu Ile Thr Glu
                405                 410                 415

Glu Glu Tyr Leu Lys Ser Met Asp Ser Phe Ala Pro Tyr Phe Lys Glu
            420                 425                 430

Val Thr Glu Glu His Trp Gln Asn Leu Val Glu Ala Tyr Arg Asn Val
        435                 440                 445

Phe Pro Glu Glu Leu Pro Ile Gly Glu Arg Leu Arg Asn Gly Val Gly
    450                 455                 460

Arg Phe Leu Gly Asp Leu Phe Phe Thr Cys Asp Leu Ala Asp Phe Thr
465                 470                 475                 480

Asn Lys Phe Val Lys Asn Gln Glu Asp Ser Val Tyr Ser Tyr Tyr Phe
                485                 490                 495

Asn Met Arg Ser Ser Ala Asn Pro Trp Pro Glu Trp Ile Gly Val Met
            500                 505                 510

His Gly Tyr Glu Ile Glu Tyr Met Phe Gly Gln Pro Phe Phe Met Pro
        515                 520                 525

Ser Val Tyr Lys Glu Glu Leu Leu Glu Leu Glu Arg Glu Phe Ser Lys
    530                 535                 540

Tyr Met Met Lys Leu Leu Ala Asp Phe Val Arg Asp Gly Lys Pro Ser
545                 550                 555                 560

Asp Asp Trp Leu Pro Tyr Thr Thr Thr Asp Arg Lys Ala Leu Val Leu
                565                 570                 575

Asp Lys Asp Ser Met Glu Gly Asn Thr Lys Tyr Val Asp Leu His Glu
            580                 585                 590

Glu Gln Cys Gln Leu Ile Lys Asp Ala His Pro Ser Pro Gly Glu Pro
        595                 600                 605

Ser Ser Thr Ser Glu
    610

<210>  31

<211>  1782

<212>  DNA

<213>  Dictyocaulus viviparus


<400>  31
tacgactcac tatagggcga attgaattta gcggccgcga attcgccctt atgttcaaac      60

tcgcctgcct ttgcgtcttt gctctgtcgg cattcgccgc aactgttgaa gaagaggaga     120

atgtgatcgt gcttacaaag gacaactttg acgaagttat taacggtcat gagtttgtgc     180

ttgccgaatt ttatgctcct tggtgcggtc actgtaaggc acttgctccg gaatatgcca     240

aagctgccac acagctgaag gaagagggtt caacgatcaa attagcaaaa ctagatgcta     300

ccgttcatgg cgatgttgct agcaagttcg aggttcgcgg gtacccaaca ctgaaactgt     360

tccgcagtgg aaagccgagt gaatacagcg gtggacgcga tgcggcatca atcattgcat 420
ggttgaagaa gaagactggt ccggtcgcga agacattaaa aactgccgat gacgtcaaat 480
cacttcaaga agaggctgat gttgttgtcg ttggatactt caagagtgtc gaaggggaga 540
aggcgaaagt ctttttggaa gttgcaagtg gagttgataa tattccattt ggaatcacct 600
ctgaagatgc tgcaaagaag cagctagaac ttaaggaaga aggtatcgtg ctattgaaga 660
agttcgacga tggacgcgca gtgtttgatg agaagctcac tgctgacaat ctcaaaacat 720
ggatccaagc caaccgacta gcccttgttt cggaattcac tcaggaaacg gcatctgtca 780
tttttggtgg agagattaaa agccataacc ttctttttgt ctccaaagag tccagcgagt 840
tcgaaaagtt agaaactgaa ttcaaaaatg ctgctagaca atttaagggc aaagttctct 900
ttgtatacat caatacggat gttgaagaca acgttcgtat aatggaattc tttggtctta 960
agaacactga cttgccagca gtacgactaa tctcattaga agaggatatg actaaattca 1020
aacctgactt tgtcgagatt aacacagaga gcattgttaa attcacccaa gcgtatttgg 1080
atggaactct caaggctcac ctcatgtccg aagagattcc tgaagattgg gataaagcac 1140
ctgtaaaagt gcttgtagga aagaacttcg accaagtagc acgggacaat acaaaaaatg 1200
ttcttgttga gttctatgct ccatggtgtg gtcactgcaa gcaacttgct ccgacttggg 1260
acaagcttgg agaaaagtat gccgaccacg agaacatcat aattgccaaa atggacgcaa 1320
ctgctaatga ggtcgaggac gtaaaagtgc aatcattccc gacaatcaag ttcttccccg 1380
caggatcaaa taaaataatc gactacactg gtgacaggac tcttgaggga ttcactaaat 1440
tccttgaaag cggtggtaag gaaggggctg gaccatcaga tgatgagaag gcagctgaag 1500
aggctgaaga cgagggtcat actgagctgt aagcatttgc tgctatctta ctggggtcgt 1560
acggtctgac gacctgttgt tgttacagtt aatttatttg ctctactaat cacccatgta 1620
cgagtgatct aaacattgcc ttcctgtata tgcgtttttt ctcttcttca attgtgatcg 1680
ttgataagtg atggtgaaat ttatttattt ttgttcagaa ctacaccact gtgttggaga 1740
actgggttta aataaatata acattttgcc aaaaaaaaaa aa 1782

<210> 32
<211> 1788
<212> DNA
<213> Dictyocaulus viviparus

<400> 32
   ctaatacgac tcactatagg gcaagcagtg gtatcaacgc agagtacgcg ggggtttaat 60
   tacccaagtt tgagggtgca ccgcggatga catttgctta tgtttttgct gtgttatttc 120
   tgtgtgcttc ggcaagcgat gtgttagagt acaccgatgc tatctttgag gactctatca 180
   agttccatga aatcgctttg gtcaaattct acgctccgtg gtgtggccac tgtaagaaaa 240
   tggctccaga atttgacaaa gcttcaacta agctgaaaag caacgatcct cccgtcgctt 300
   taattaaggt tgattgcact gtcgaaaaat ccacatgtga caaatatggc gtcaaaggtt 360
   tcccaacgtt aaaaatcttt cgctttggat cggaagctca ggcatatgag ggacctcgtg 420
   atgcggacgg tatcgtcaag tatatgcgtg ggcaagctgg tccttctgcc agagagataa 480
   aatctatcaa cgagttcaag aaagctatca gtggagatga aaatattgtt attggcttct 540
   tcgagaatga gagcaagttg aaagattcat tcctcaaagt tgctgacact gaaagagacc 600
   gtttccagtt tgcatacact tctgatcgtt ctgtactcaa agaaacggga tataacgatg 660
   atatcgttgt gttcacgccg aagcagctcc ataataagtt tgatcctaat gaattcaaat 720
   atgatggcaa ctatgatacg gacaaaatca agaatttcct cattcatgaa accgtcggaa 780
   tggctgggat tcgcactcat ggaaatttgt tccaatttga acagaagcct cttgtcattg 840
   tatattacaa tgtcgactat ttgaaagatc ctaaaggttc gaattattgg cgtaatcgtg 900
   tattgaaggt ggccaaagat tacaagcgca aggttcattt tgctgtatca aataaagaag 960
   aattttcatc agaagttgat caaaatggac tttccttgcg taaagactca gataaaccta 1020
   tcgttgcagc tgtgacaaat gaaggcaaat ttcctatgga taacgaattc agcgttgaca 1080
   atcttaagac cttcgttgaa gatttgattg ctggtagact tgaaccttac atgaaatctg 1140
   aaccaattcc tgagaataca ggagcattaa aggttgcagt tgccaagaat ttcaaagagt 1200
   tagtactgaa cgccaaaaaa gatgttcttg tagaattttt tacgctccgt ggtgcggaca 1260
   ctgcaaggcg gttggctccg aaatacgaag aacttgccga gaagttagta gatgaagatg 1320
   ttctcattgt aaaaatggat gcccccgcta atgatgtacc tcccttgttt gaagtcaatg 1380
   gatttcctac aatatactgg ttgccaaaga acaagaaggg gtccccagtt ccttacagtg 1440
   gtggccgaga agtggacgac tttatctcct tcatagctaa gcactcaacc gatggcctca 1500
   agcaatattc acgtgatgga aagaagaaga aggcagagtt gtaattttgt aagcgttcat 1560
   tgttgaagaa gcttttgcat ggtgtttaca agaacatcat taatatgata caatgcaaca 1620
   tttgcacttt tttccactgt tatttattat ttcgaagggt ggtcttcacg gttgtttgtt 1680
   gttacatagg cttatgatgt gccttttatg gttttcacat aattttattg tagcaagtat 1740
   ttatcagaag tcaataaatc gttgtttctg caaaaaaaaa aaaaaaaa 1788

<210> 33
<211> 493
<212> PRT
<213> Dictyocaulus viviparus

<400> 33

Met Phe Lys Leu Ala Cys Leu Cys Val Phe Ala Leu Ser Ala Phe Ala
1               5                   10                  15

Ala Thr Val Glu Glu Glu Glu Asn Val Ile Val Leu Thr Lys Asp Asn

20                          25                          30

Phe Asp Glu Val Ile Asn Gly His Glu Phe Val Leu Ala Glu Phe Tyr
        35                  40                  45

Ala Pro Trp Cys Gly His Cys Lys Ala Leu Ala Pro Glu Tyr Ala Lys
    50                  55                  60

Ala Ala Thr Gln Leu Lys Glu Glu Gly Ser Thr Ile Lys Leu Ala Lys
65                  70                  75                  80

Leu Asp Ala Thr Val His Gly Asp Val Ala Ser Lys Phe Glu Val Arg
                85                  90                  95

Gly Tyr Pro Thr Leu Lys Leu Phe Arg Ser Gly Lys Pro Ser Glu Tyr
            100                 105                 110

Ser Gly Gly Arg Asp Ala Ala Ser Ile Ile Ala Trp Leu Lys Lys Lys
        115                 120                 125

Thr Gly Pro Val Ala Lys Thr Leu Lys Thr Ala Asp Asp Val Lys Ser
    130                 135                 140

Leu Gln Glu Glu Ala Asp Val Val Val Val Gly Tyr Phe Lys Ser Val
145                 150                 155                 160

Glu Gly Glu Lys Ala Lys Val Phe Leu Glu Val Ala Ser Gly Val Asp
                165                 170                 175

Asn Ile Pro Phe Gly Ile Thr Ser Glu Asp Ala Ala Lys Lys Gln Leu
            180                 185                 190

Glu Leu Lys Glu Glu Gly Ile Val Leu Leu Lys Lys Phe Asp Asp Gly
        195                 200                 205

Arg Ala Val Phe Asp Glu Lys Leu Thr Ala Asp Asn Leu Lys Thr Trp
    210                 215                 220

Ile Gln Ala Asn Arg Leu Ala Leu Val Ser Glu Phe Thr Gln Glu Thr
225                 230                 235                 240

Ala Ser Val Ile Phe Gly Gly Glu Ile Lys Ser His Asn Leu Leu Phe
                245                 250                 255

Val Ser Lys Glu Ser Ser Glu Phe Glu Lys Leu Glu Thr Glu Phe Lys
            260                 265                 270

Asn Ala Ala Arg Gln Phe Lys Gly Lys Val Leu Phe Val Tyr Ile Asn
        275                 280                 285

Thr Asp Val Glu Asp Asn Val Arg Ile Met Glu Phe Phe Gly Leu Lys
    290                 295                 300

Asn Thr Asp Leu Pro Ala Val Arg Leu Ile Ser Leu Glu Glu Asp Met
305                 310                 315                 320

Thr Lys Phe Lys Pro Asp Phe Val Glu Ile Asn Thr Glu Ser Ile Val
                325                 330                 335

Lys Phe Thr Gln Ala Tyr Leu Asp Gly Thr Leu Lys Ala His Leu Met
            340                 345                 350

Ser Glu Glu Ile Pro Glu Asp Trp Asp Lys Ala Pro Val Lys Val Leu
        355                 360                 365

Val Gly Lys Asn Phe Asp Gln Val Ala Arg Asp Asn Thr Lys Asn Val
    370                 375                 380

Leu Val Glu Phe Tyr Ala Pro Trp Cys Gly His Cys Lys Gln Leu Ala
385                 390                 395                 400

Pro Thr Trp Asp Lys Leu Gly Glu Lys Tyr Ala Asp His Glu Asn Ile
                405                 410                 415

Ile Ile Ala Lys Met Asp Ala Thr Ala Asn Glu Val Glu Asp Val Lys
            420                 425                 430

Val Gln Ser Phe Pro Thr Ile Lys Phe Phe Pro Ala Gly Ser Asn Lys
        435                 440                 445

Ile Ile Asp Tyr Thr Gly Asp Arg Thr Leu Glu Gly Phe Thr Lys Phe
    450                 455                 460

Leu Glu Ser Gly Gly Lys Glu Gly Ala Gly Pro Ser Asp Asp Glu Lys
465                 470                 475                 480

Ala Ala Glu Glu Ala Glu Asp Glu Gly His Thr Glu Leu
                485                 490

<210>  34

<211>  485

<212>  PRT

<213>  Dictyocaulus viviparus

<400>  34

Met Thr Phe Ala Tyr Val Phe Ala Val Leu Phe Leu Cys Ala Ser Ala
1               5                   10                  15

Ser Asp Val Leu Glu Tyr Thr Asp Ala Ile Phe Glu Asp Ser Ile Lys
            20                  25                  30

Phe His Glu Ile Ala Leu Val Lys Phe Tyr Ala Pro Trp Cys Gly His
        35                  40                  45

Cys Lys Lys Met Ala Pro Glu Phe Asp Lys Ala Ser Thr Lys Leu Lys
    50                  55                  60

Ser Asn Asp Pro Pro Val Ala Leu Ile Lys Val Asp Cys Thr Val Glu
65                  70                  75                  80

Lys Ser Thr Cys Asp Lys Tyr Gly Val Lys Gly Phe Pro Thr Leu Lys
                85                  90                  95

Ile Phe Arg Phe Gly Ser Glu Ala Gln Ala Tyr Glu Gly Pro Arg Asp
            100                 105                 110

Ala Asp Gly Ile Val Lys Tyr Met Arg Gly Gln Ala Gly Pro Ser Ala
        115                 120                 125

Arg Glu Ile Lys Ser Ile Asn Glu Phe Lys Lys Ala Ile Ser Gly Asp
    130                 135                 140

Glu Asn Ile Val Ile Gly Phe Phe Glu Asn Glu Ser Lys Leu Lys Asp
145                 150                 155                 160

Ser Phe Leu Lys Val Ala Asp Thr Glu Arg Asp Arg Phe Gln Phe Ala
                165                 170                 175

Tyr Thr Ser Asp Arg Ser Val Leu Lys Glu Thr Gly Tyr Asn Asp Asp
            180                 185                 190

Ile Val Val Phe Thr Pro Lys Gln Leu His Asn Lys Phe Asp Pro Asn
        195                 200                 205

Glu Phe Lys Tyr Asp Gly Asn Tyr Asp Thr Asp Lys Ile Lys Asn Phe
    210                 215                 220

Leu Ile His Glu Thr Val Gly Met Ala Gly Ile Arg Thr His Gly Asn
225                 230                 235                 240

Leu Phe Gln Phe Glu Gln Lys Pro Leu Val Ile Val Tyr Tyr Asn Val
                245                 250                 255

Asp Tyr Leu Lys Asp Pro Lys Gly Ser Asn Tyr Trp Arg Asn Arg Val
            260                 265                 270

Leu Lys Val Ala Lys Asp Tyr Lys Arg Lys Val His Phe Ala Val Ser
        275                 280                 285

Asn Lys Glu Glu Phe Ser Ser Glu Val Asp Gln Asn Gly Leu Ser Leu
    290                 295                 300

Arg Lys Asp Ser Asp Lys Pro Ile Val Ala Ala Val Thr Asn Glu Gly
305                 310                 315                 320

Lys Phe Pro Met Asp Asn Glu Phe Ser Val Asp Asn Leu Lys Thr Phe
                325                 330                 335

Val Glu Asp Leu Ile Ala Gly Arg Leu Glu Pro Tyr Met Lys Ser Glu
            340                 345                 350

Pro Ile Pro Glu Asn Thr Gly Ala Leu Lys Val Ala Val Ala Lys Asn
        355                 360                 365

Phe Lys Glu Leu Val Leu Asn Ala Lys Lys Asp Val Leu Val Glu Phe
    370                 375                 380

Phe Thr Leu Arg Gly Ala Asp Thr Ala Arg Arg Leu Ala Pro Lys Tyr
385                 390                 395                 400

Glu Glu Leu Ala Glu Lys Leu Val Asp Glu Asp Val Leu Ile Val Lys
                405                 410                 415

Met Asp Ala Pro Ala Asn Asp Val Pro Pro Leu Phe Glu Val Asn Gly
            420                 425                 430

Phe Pro Thr Ile Tyr Trp Leu Pro Lys Asn Lys Lys Gly Ser Pro Val
        435                 440                 445

Pro Tyr Ser Gly Gly Arg Glu Val Asp Asp Phe Ile Ser Phe Ile Ala
    450                 455                 460

Lys His Ser Thr Asp Gly Leu Lys Gln Tyr Ser Arg Asp Gly Lys Lys
465                 470                 475                 480

Lys Lys Ala Glu Leu
                485

<210>  35

<211>  22

<212>  DNA

<213>  artificial sequence

<220>

<223>  primer

<400>  35
ggatccgagt cagcagacgg tg                                                22

<210>  36

<211>  33

<212>  DNA
<213>  artificial sequence

<220>
<223>  primer
<400>  36
gcggccgcaa caacagagta gaattaggaa gta                                    33

<210>  37
<211>  24
<212>  DNA
<213>  artificial sequence

<220>
<223>  primer
<400>  37
ggatccatac tccacatttc actc                                              24

<210>  38
<211>  30
<212>  DNA
<213>  artificial sequence

<220>
<223>  primer
<400>  38
cttaagccaa ttccaagcgt ttagtttaga                                        30

<210>  39
<211>  23
<212>  DNA
<213>  artificial sequence

<220>
<223>  primer
<400>  39
ggatcctctg ctaacttgta cag                                               23

<210> 40

<211> 30

<212> DNA

<213> artificial sequence

<220>

<223> primer

<400> 40
gcggccgctt ttgcggtcac cttagaaatc 30